# EUROPEAN PATENT APPLICATION

(11) **EP 4 635 488 A1**
(43) Date of publication of application: **22.10.2025**
(21) Application number: 22968596.1
(22) Date of filing: 13.12.2022
(51) Int. Cl.: A61K 31/167, A61P 19/00, A61P 19/08, A23L 33/10

(54) **PHARMACEUTICAL COMPOSITION FOR PREVENTING OR TREATING BONE DISEASES**

(71) Applicant: Industry Foundation of Chonnam National University, Gwangju 61186 (KR)
(72) Inventor: LEE, Tae Hoon, Gwangju 61033 (KR); LEE, Sun Woo, Gwangju 61026 (KR); PARK, Sang Wook, Gwangju 61186 (KR); CHEN, Zhihao, Nanyang City, Henan 474450 (CN); RAJAMANICKAM, Karthik Rajan, Salem (TK), Tamil Nadu, 636116 (IN)
(74) Representative: Dompatent Partnerschaft von Patent- und Rechtsanwälten mbB
(86) International application number: PCT/KR2022/020245
(87) International publication number: WO 2024/128335

(57) **Abstract**

The present invention relates to a composition which has the effect of inhibiting osteoclast differentiation and/or generation due to including a specific compound, and thus can exhibit excellent medicinal effect and health functional properties for bone diseases including osteoporosis and the like.

## Description

### [Technical Field]

The present invention relates to a pharmaceutical composition and health functional foods for prevention or treatment of bone diseases, and specifically to a pharmaceutical composition and health functional foods for prevention or treatment of bone diseases having the effect of inhibiting osteoclast differentiation and/or production.

### [Background Art]

Bone tissue remodeling is regulated by bone resorption through osteoclasts, whereas bone formation is regulated using osteoblasts. Osteoclasts are large, multinucleated cells that resorb bone and cause bone loss. In healthy bone, the dynamics between bone resorption and bone formation are integrated and balanced.

However, when bone resorption exceeds bone formation, common bone diseases such as osteoporosis may occur. The most common and available treatment for osteoporosis is the use of antiresorptive drugs such as bisphosphonates and receptor activators of nuclear factor kappa-B (NF-κ ligand) (RANKL) antibodies.

However, the use of bisphosphonate drugs is associated with side effects such as osteonecrosis of the jaw, hypocalcemia, and gastrointestinal disorders. Accordingly, there is a need to develop novel and effective treatments for osteoporosis.

### [Summary of Invention]

### [Problems to be Solved by Invention]

An object of the present invention is to provide a pharmaceutical composition for preventing or treating bone diseases.

Another object of the present invention is to provide a health functional food for preventing or improving bone diseases.

### [Means for Solving Problems]

1. A pharmaceutical composition for preventing or treating bone disease caused by hyper-differentiation of osteoclasts, comprising a compound represented by Formula 1 below or a pharmaceutically acceptable salt thereof: 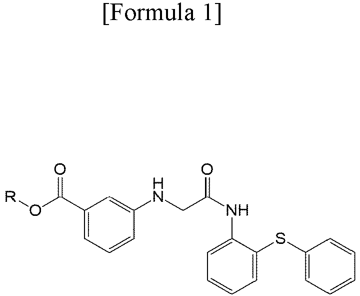 wherein R is a C₁₋₅ alkyl group.
2. The pharmaceutical composition according to the above 1, wherein R is a methyl group.
3. The pharmaceutical composition according to the above 1, wherein the bone disease is at least one selected from the group consisting of a fracture, osteoporosis, rheumatoid arthritis, periodontitis, Paget's disease, osteomalacia, osteopenia, bone atrophy, osteoarthritis, and avascular femoral necrosis.
4. A health functional food for preventing or improving bone disease caused by hyper-differentiation of osteoclasts, comprising a compound represented by Formula 1 below or a pharmaceutically acceptable salt thereof: 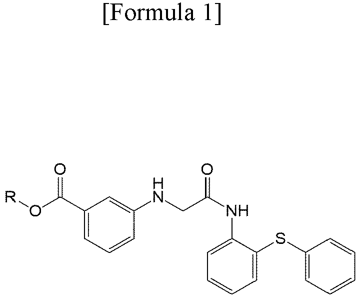 wherein R is a C₁₋₅ alkyl group.
5. The health functional food according to the above 4, wherein the bone disease is at least one selected from the group consisting of a fracture, osteoporosis, rheumatoid arthritis, periodontitis, Paget's disease, osteomalacia, osteopenia, bone atrophy, osteoarthritis, and avascular femoral necrosis.

### [Advantageous effects]

The composition of the present invention has the effect of inhibiting osteoclast differentiation and/or production, thereby exhibiting excellent pharmacological efficacy and health functionality for bone diseases.

### [Brief Description of Drawings]

FIGS. 1A-D and 2A-B schematically illustrate the synthetic route of 25-KR-# derivative in the examples of the present application ("present examples").
FIGS. 3A-D show the results of an experiment in which 25-KR-03 of the present examples attenuated RANKL-induced osteoclast formation in vitro. * *p <0.05, ** p* <0.01, and **** p* <0.001 vs. vehicle-treated Con (0 µM); "Con": M-CSF and RANKL treatment; "M": M-CSF treatment; scale bar = 200 µm.
FIGS. 4A-D show the experimental results of the effect of 25-KR-03 in the present examples on inhibiting F-actin ring formation and bone resorption induced by RANKL/M-CSF. Bars: mean ± SD; ** p* <0.05, *** p* <0.01 and **** p* <0.001 vs. vehicle-treated control (Con, 0 µM 25-KR-03); "M": M-CSF treatment; "Con": M-CSF and RANKL treatment; scale bar = 200 µm.
FIGS. 5A-F show the results of an experiment in which 25-KR-03 of the present examples inhibited the expression of osteoclast-specific genes induced by RANKL/M-CSF. Bars: mean ± SD; * *p* <0.05, ** *p* <0.01, and *** *p* <0.001 vs. vehicle-treated control (Con, 0 µM).
FIGS. 6A-C show the results of an experiment in which 25-KR-03 of the present examples suppressed osteoclastogenesis induced by RANKL by reducing NFATc1 nuclear translocation.
FIGS. 7A-B show the results of an experiment in which 25-KR-03 of the present examples attenuated hRANKL/M-CSF-induced osteoclastogenesis using human IPSC-derived macrophages.
FIGS. 8A-E show the results of an experiment in which 25-KR-03 of the present examples prevented bone loss in ovariectomized mice with osteoporosis.
FIG. 9 shows photographs of uterine tissues of each sham, OVX, 25-KR-03, and PMSA-3-Ac experimental group in the present examples.
FIG. 10 is a schematic diagram of the ADME analysis results for 25-KR-03 in the present examples.

### [Mode for Carrying out Invention]

Hereinafter, the present invention will be described in detail.

The present invention relates to a pharmaceutical composition for preventing or treating bone disease caused by hyper-differentiation of osteoclasts, including a compound represented by Formula 1 below or a pharmaceutically acceptable salt thereof.

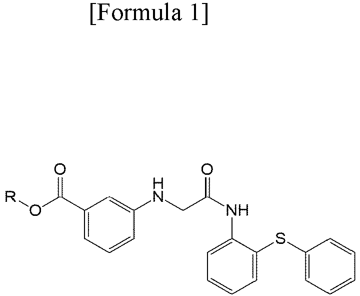

In the above formula, R may be C₁₋₅ alkyl.

The C₁₋₅ alkyl may be a straight or branched, saturated, monovalent hydrocarbon group having 1, 2, 3, 4 or 5 carbon atoms, for example, methyl, ethyl, n-propyl, isopropyl, n-butyl, tert-butyl, n-pentyl, n-hexyl or isomers thereof.

Specifically, R may be methyl.

The compounds included in the present invention may be derived from nature or may be synthesized using known chemical synthesis methods.

The salt of the compound may be a pharmaceutically acceptable salt.

A useful salt is, for example, an acid addition salt formed by a free acid. Acid addition salts may include: inorganic acids such as hydrochloric acid, nitric acid, phosphoric acid, sulfuric acid, hydrobromic acid, hydroiodic acid, nitrous acid or phosphorous acid; and non-toxic organic acids such as aliphatic mono and dicarboxylates, phenyl-substituted alkanoates, hydroxy alkanoates and alkane dioates, aromatic acids, aliphatic and aromatic sulfonic acids. These pharmaceutically or sitologically non-toxic salts may include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate propionate, decanoate, caprylate, acrylate, formate, isobutyrate, caprate, heptanoate, propylate, oxalate, malonate, succinate, suberate, sebacate, fumarate, maleate, butyne-1,4-dioate, hexane-1,6-dioate, benzoate, chlorobenzoate, methyl benzoate, dinitro benzoate, hydroxybenzoate, methoxybenzoate, phthalate, terephthalate, benzene sulfonate, toluene sulfonate, chlorobenzene sulfonate, xylene sulfonate, phenylacetate, phenylpropionate, phenylbutyrate, citrate, lactate, β_hydroxybutyrate, glycolate, maleate, tartrate methane sulfonate, propane sulfonate, naphthalene-1-sulfonate, naphthalene-2-sulfonate or mandelate.

The acid addition salt may be prepared by a conventional method, for example, by dissolving the compound represented by Formula 1 in an excess of acid aqueous solution, and precipitating this salt in a hydratable organic solvent, such as methane, ethane, acetone, or acetonitrile. Alternatively, it may also be prepared by heating the compound represented by Formula 1 and an acid or alcohol in water, then evaporating the mixture to dryness, or suction-filtering the precipitated salt.

Further, a pharmaceutically acceptable metal salt may be prepared using a base. The alkali metal or alkaline earth metal salt is obtained, for example, by dissolving the compound in an excess of alkali metal hydroxide or alkaline earth metal hydroxide solution, filtering the insoluble compound salt, and evaporating and drying the filtrate. At this time, it is pharmaceutically appropriate to produce sodium, potassium or calcium salts as metal salts. In addition, the corresponding silver salt is obtained by reacting an alkali metal or alkaline earth metal salt with an appropriate silver salt (for example, silver nitrate).

Bone diseases that can be prevented or treated by the pharmaceutical composition of the present invention may be caused by activity imbalance between osteoblasts and osteoclasts.

Because bone is a living tissue, old bone is constantly destroyed and goes through a remodeling process to create new bone. During this process, osteoclasts destroy old and unnecessary bone tissue to release calcium into the bloodstream to help maintain body functions, and osteoblasts play a role in regenerating destroyed bones. This process continues 24 hours a day, and approximately 10 to 30% of an adult's bones are rebuilt in this way each year. Therefore, the balance between osteoclasts and osteoblasts is very important, and this balance is regulated by various hormones and other body chemical components.

Specifically, the bone disease may be caused by hyper-differentiation of osteoclasts or a decrease in osteoblast activity, but is not limited thereto. Preferably, the bone disease may be caused by hyper-differentiation of osteoclasts.

When osteoclasts become hyperdifferentiated, the number of osteoclasts increases abnormally, resulting in excessive bone resorption, which may lead to loss of bone within the bone and lower bone density. For example, various diseases such as osteoporosis, osteomalacia, osteopenia, bone atrophy, and periodontitis may occur.

The bone diseases may include fractures, osteoporosis, rheumatoid arthritis, periodontitis, Paget's disease, osteomalacia, osteopenia, bone atrophy, osteoarthritis or avascular femoral necrosis, bone defects, fractures, osteoporotic fractures, diabetic fractures, nonunion fractures, osteogenesis imperfecta, osteomalacia fracture, osteogenesis disorder, degenerative bone disease, malocclusion, bone union disorder, pseudarthrosis, osteonecrosis, osteoarthritis, bone tumor, bone cancer, etc., but are not limited thereto. Preferably, the bone disease may be fracture, osteoporosis, rheumatoid arthritis, periodontitis, Paget's disease, osteomalacia, osteopenia, bone atrophy, osteoarthritis, or avascular femoral osteonecrosis, but is not limited thereto.

The periodontitis is an inflammatory reaction caused by the defense action of immune cells against periapical bacterial infection. Neutrophils, which are mainly involved in periodontitis, secrete prostaglandins as an inflammatory mediator. Osteoclasts absorbing bone tissues are activated by cell signaling substances such as prostaglandin, and loss of alveolar bone is observed around the inflamed area. Further, chronic periodontitis indicates persistent inflammation of the periapical part of the periodontium and alveolar bone erosion. If periodontitis worsens and the tooth cannot be saved, the tooth is extracted and an implant should be performed. To prevent this, relief and treatment of chronic periodontitis may be predicted by administering antibiotics to reduce the number of cells causing infection in the early stages of periodontitis and administering osteoclast inhibitors that can overcome osteoclast activation caused by inflammatory mediators.

The term "prevention" as used herein may mean any action to inhibit or delay bone disease.

The term "treatment" as used herein may mean any action that improves or beneficially changes the symptoms of an individual afflicted with or suspected of having a bone disease.

The pharmaceutical composition according to the present invention may include the active ingredient alone or may be provided as a pharmaceutical composition including one or more pharmaceutically acceptable carriers, excipients, or diluents as well as the active ingredient.

The term "pharmaceutically acceptable" as used herein may mean that the composition exhibits non-toxic properties to cells or humans exposed to the composition.

Furthermore, the pharmaceutical composition of the present invention may be provided by mixing the same with conventionally known substances for treating bone diseases. That is, the pharmaceutical composition of the present invention may be administered in combination with a known compound that has the effect of preventing or treating bone disease.

The term "administration" as used herein means introducing a predetermined substance into an individual by an appropriate method, and the term "individual" as used herein refers to all animals such as rats, mice, livestock, etc., including humans who have or may develop bone disease. As a specific example, it may be a mammal, including humans.

If necessary, the pharmaceutical composition of the present invention may additionally include known anti-bone disease compounds.

These anti-bone disease compounds may include, for example, cinchonine, brown mealworm extract, aloe-imodine and omega-3 fatty acids, arteanuin B, indole-2-carboxylate derivatives, euphorbia factor L1, skullcap flavone derivatives, fraxinelone, etc., but are not limited thereto.

In the present invention, the route of administration of the pharmaceutical composition may include, but is not limited to, oral, intravenous, intramuscular, intraarterial, intramedullary, intrathecal, intracardiac, transdermal, subcutaneous, intraperitoneal, intranasal, enteral, topical, sublingual or rectal route.

The composition of the present invention may be administered orally or parenterally, and when administered parenterally, it is preferable to select any suitable administration method among external application through the skin, or intraperitoneal injection, intrarectal injection, subcutaneous injection, intravenous injection, intramuscular injection, or intrathoracic injection, which is not limited thereto.

A preferred dosage of the composition of the present invention varies depending on the patient's condition and weight, degree of disease, drug form, administration route and period, but may be appropriately selected by a person skilled in the art. However, for desirable effects, the composition is preferably administered at 0.01 to 1000 mg/kg/day, preferably 0.1 to 500 mg/kg/day, but is not limited thereto. The above administration may be performed once a day, or several times. The above dosage does not limit the scope of the present invention in any way.

When formulated, it is prepared using diluents or excipients such as commonly used fillers, extenders, binders, wetting agents, disintegrants, and surfactants. Solid preparations for oral administration may include tablets, pills, powders, granules, capsules, etc. These solid preparations may include the extract with at least one excipient, such as starch, calcium carbonate, and sucrose. Alternatively, it is prepared by mixing lactose, gelatin, etc. In addition to simple excipients, lubricants such as magnesium stearate and talc are also used. Liquid preparations for oral use may include suspensions, oral solutions, emulsions, syrups, etc. In addition to the commonly used simple diluents such as water and liquid paraffin, various excipients such as wetting agents, sweeteners, fragrances, and preservatives may be included. Preparations for parenteral administration may include sterile aqueous solutions, non-aqueous solutions, suspensions, emulsions, lyophilized preparations, and suppositories. Non-aqueous solvents and suspensions may include propylene glycol, polyethylene glycol, vegetable oil such as olive oil, and injectable ester such as ethyl oleate. As a base for suppositories, witepsol, macrogol, tween 61, cacao, laurel, glycerogeratin, etc. may be used.

Further, the present invention provides a health functional food for preventing or improving bone disease caused by hyper-differentiation of osteoclasts including a compound represented by Formula 1 below or a sitologically acceptable salt thereof.

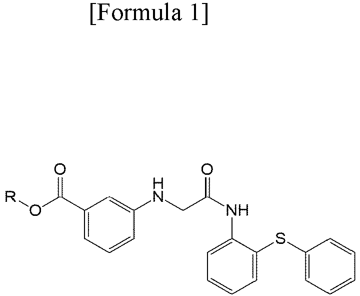

In the above formula, R may be C₁₋₅ alkyl, and specifically may be methyl.

The detailed description of Formula 1, specific examples, and effects are the same as described above, and therefore will not be described to avoid duplication.

The salt of the compound represented by Formula 1 may be a sitologically acceptable salt, and specific examples may be within the above-mentioned range, but are not limited thereto.

The bone disease may be within the range described above, but is not limited thereto.

The health functional food of the present invention may be formulated into one selected from the group consisting of tablets, pills, powders, granules, powders, capsules, and liquid formulations, by further including one or more of carriers, diluents, excipients, and additives. Foods to which the extract of the present invention is added may include various foods, powders, granules, tablets, capsules, syrups, beverages, gum, tea, vitamin complexes, health functional foods, etc.

Additives further included in the present invention may include one or more component selected from the group consisting of natural carbohydrates, flavors, nutrients, vitamins, minerals (electrolytes), flavors (synthetic flavors, natural flavors, etc.), colorants, fillers (cheese, chocolate, etc.), pectic acid and its salts, alginic acid and its salts, organic acids, protective colloidal thickeners, pH adjusters, stabilizers, preservatives, antioxidants, glycerin, alcohol, carbonating agents and pulp.

Examples of the above-mentioned natural carbohydrates may include: monosaccharides such as glucose, fructose, etc.; disaccharides such as maltose, sucrose, etc.; and polysaccharides, such as common sugars, for example, dextrin and cyclodextrin, and sugar alcohols such as xylitol, sorbitol, and erythritol. As the flavoring agent, natural flavoring agents (thaumatin, stevia extract (e.g., rebaudioside A, glycyrrhizin, etc.)) and synthetic flavoring agents (saccharin, aspartame, etc.) may be advantageously used.

In addition to the above, the health functional food of the present invention may contain various nutrients, vitamins, minerals (electrolytes), flavoring agents such as synthetic and natural flavors, colorants and thickening agents (cheese, chocolate, etc.), pectic acid and its salts, and alginic acid and its salts, organic acids, protective colloidal thickeners, pH adjusters, stabilizers, preservatives, glycerin, alcohol, carbonating agents used in carbonated beverages, etc. In addition, the composition according to the present invention may contain pulp for the production of natural fruit juice and vegetable drinks. These ingredients may be used independently or in combination.

Specific examples of the carriers, excipients, diluents and additives may include, but are not limited to, preferably one or more selected from the group consisting of lactose, dextrose, sucrose, sorbitol, mannitol, erythritol, starch, gum acacia, calcium phosphate, alginate, gelatin, calcium phosphate, calcium silicate, microcrystalline cellulose, polyvinylpyrrolidone, cellulose, polyvinylpyrrolidone, methylcellulose, water, sugar syrup, methylcellulose, methyl hydroxybenzoate, propyl hydroxybenzoate, talc, magnesium stearate and mineral oil.

When formulating the health functional food of the present invention, it may be prepared using commonly used diluents or excipients such as fillers, extenders, binders, wetting agents, disintegrants, and surfactants.

Hereinafter, the present invention will be described in detail with reference to examples. However, the following examples are provided only to make the present invention easier to understand, and the content of the present invention is not limited by the following examples.

### Experimental Method

### 1. Chemical analysis method

All reagents were purchased and used without additional purification. ¹H-NMR spectra were recorded in dimethylsulfoxide-D6 (DMSO-_{d6}) on a 400 MHz, 500 MHz and 600 MHz NMR spectrometer, and the data is reported as follows: chemical shift, multiplicity (s = singlet, d = doublet, t = triplet, dd = doublet of doublets, dt = doublet of triplets, ddd = doublet of doublets in doublets, m = multiplet), coupling constant (Hz) and integration. ¹³C-NMR spectra were recorded in DMSO-d₆ on a 101 MHz, 126 MHz or 151 MHz NMR spectrometer and at resonance (δ) is expressed in ppm. High-resolution mass spectra were recorded on a Time-of-Flight mass spectrometer. 400 MHz ¹H-NMR was recorded on JEOL, ECZ-400R. 500 MHz 1 H-NMR was recorded on an Agilent-ProPulse NMR spectrometer. Furthermore, 600 MHz 1 H-NMR was recorded on an Agilent-VNMRS 600 (20K cryoprobe) NMR spectrometer.

### 2. Synthesis of compounds

### 2.1. Synthesis of intermediates 25-KR-01 and 25-KR-02

Commercially available 3-nitrobenzoic acid (1) (5.00 g, 29.9 mmol) was dissolved in dichloromethane (CH₂Cl₂) (50 mL), and N,N- dimethylformamide (DMF) was added thereto (0.23 mL, 2.99 mmol). Afterwards, the reaction mixture was cooled to 0 °C, the temperature was raised to room temperature (rt) upon addition, and oxalyl chloride (3.0 mL, 35.9 mmol) was added. After stirring for 4 hours, the reaction mixture was evaporated under reduced pressure and co-distilled twice with toluene (25 mL × 2) to remove excess oxalyl chloride, followed by further evaporating to dryness to obtain light yellow oil **2** in a yield of 99%.

The above product **2** dissolved in CH₂Cl₂ (25 mL) was added dropwise to a mixture of methanol (2.4 mL, 59.8 mmol) and triethylamine (8.3 mL, 59.8 mmol) in CH₂Cl₂ (25 mL) at room temperature, stirred for 5 hours, and analyzed by thin-layer chromatography (TLC). The mixture was diluted with CH₂Cl₂ and washed with 1N aqueous hydrochloric acid (HCl) solution, saturated sodium bicarbonate and brine solution (NaCl). The organic phase was dried over magnesium sulfate (MgSO₄), filtered, and concentrated. The residue was purified by flash column chromatography (n-hexane/ethyl acetate [EtOAc] 9:1) to obtain light yellow oil **3** (5.25 g, 29.00 mmol) in a yield of 97%.

Zinc (5.69 g, 87 mmol) and acetic acid (16.6 mL, 290.0 mmol) were added dropwise to a mixture of the above product **3** (5.3 g, 29.00 mmol) in ethanol:water (9:1; 53 mL) at room temperature and stirred for 10 minutes. After 4 hours, the reaction was monitored by TLC analysis. The reactant was filtered through Celite and washed with excess ethanol. The filtrate was evaporated to dryness to obtain a residue. Then, EtOAc (50 mL) was added thereto, and the mixture was washed with saturated sodium bicarbonate and brine solution. The organic phase was dried over MgSO₄, filtered and concentrated. The crude material was purified by flash column chromatography (n-hexane/EtOAc 7:3) to obtain light yellow oil **4** (4.16 g, 27.55 mmol) in a yield of 95%.

The above mixture **4** (4.16 g, 27.55 mmol) was dissolved in CH₂Cl₂ (126 mL), pyridine (4.44 mL, 55.10 mmol) was added thereto, then cooled to 0 °C. Mesyl chloride (2.56 mL, 33.06 mmol) was added to the mixture, the temperature was raised to room temperature and stirred for 12 hours, and then, the completion of reaction was monitored by TLC. The reactant was washed with 1N aqueous HCl solution, saturated sodium bicarbonate and brine solution. The organic phase was dried over MgSO₄, filtered, and concentrated to obtain a crude residue. Then, 20 mL (n-hexane/EtOAc 9:1) was added thereto, and the mixture was stirred for 10 min, filtered, followed by washing with 10 mL of distilled water. The same mixture was dried to obtain yellow solid 5 (5.87 g, 25.62 mmol) in a yield of 93%.

The above mixture **5** (688 mg, 3.0 mmol) and compound **8** (1.26 g, 3.9 mmol), as well as *N, N-* diisopropylethylamine (DIPEA) (1.04 mL, 6.0 mmol) were dissolved in acetonitrile (ACN) (6.9 mL), followed by stirring the mixture at 80 °C for 12 hours. The reaction mixture was diluted with EtOAc and washed with 1N aqueous HCl solution, saturated sodium bicarbonate and brine solution. The organic phase was dried over MgSO₄, filtered and concentrated. The residue was purified by flash column chromatography (n-hexane/EtOAc 7:3) to obtain yellow oil **25-KR-01** (1.31 g, 2.79 mmol, 93% yield).

Potassium hydroxide (KOH; 292 mg, 5.2 mmol) dissolved in methanol (6.0 mL) was added to the above **25-KR-01** (1.22 g, 2.6 mmol), and stirred at 35 °C until solids were formed. The mixture was diluted with water (30 mL) and then washed with diethyl ether (Et₂O; 30 mL). The pH of the aqueous layer was adjusted to 4 using 6N HCl. The aqueous mixture was extracted with Et₂O (30 mL × 2) and the organic phase was dried over MgSO₄, filtered and concentrated to obtain yellow solid **25-KR-02** (1.01 g, 2.21 mmol, 85% yield). The resulting solid was then used for the next step without additional purification.

### Methyl 3-(N-(2-oxo-2-((2-(phenylthio)phenyl)amino)ethyl)methylsulfonamido) benzoate (25-KR-01)

Light yellow oil (1.31 g), 93% yield; ¹H NMR (500MHz, DMSO-d₆) δ 9.74 (s, 1H), 8.09 (t, *J* = 1.9Hz, 1H), 7.88 (dt, *J* = 7.8, 1.3Hz, 1H), 7.75 (d, *J* = 8.0Hz, 1H), 7.70 (ddd, *J =* 8.1, 2.3, 1.1Hz, 1H), 7.51 (t, *J =* 7.9Hz, 1H), 7.39 - 7.34 (m, 2H), 7.31 - 7.28 (m, 2H), 7.24 - 7.16 (m, 4H), 4.54 (s, 2H), 3.84 (s, 3H), 3.13 (s, 3H); ¹³C NMR (126 MHz, DMSO- d₆) *δ* 167.5, 166.0, 141.0, 138.1, 135.3, 134.3, 132.5, 131.2, 130.2, 130.0, 129.9, 129.5, 128.6, 128.5, 127.5, 126.6, 124.8, 524.3, 39.08, 59.08, 59.08 HRMS (FD-TOF) m/z: [M]+ C₂₃H₂₂N₂O₅S₂, calculated, found, 470.0988.

### 3-(N-(2-oxo-2-((2-(phenylthio)phenyl)amino)ethyl)methylsulfonamido)benzoic acid (25-KR-02)

Light yellow solid (1.01 g), yield 85%; 1H NMR (500 MHz, DMSO-d₆) δ 9.75 (s, 1H), 8.07 - 8.06 (m, 1H), 7.86 (dt, J = 7.9, 1.2Hz, 1H), 7.82 (d, J = 7.7Hz, 1H), 7.54 (ddd, *J* = 8.0, 2.3, 1.2Hz, 1H), 7.41 - 7.36 (m, 3H), 7.31 - 7.27 (m, 2H), 7.23 - 7.21 (m, 1H), 7.20 - 7.17 (m, 3H), 4.51 (s, 2H), 3.12 (s, 3H); ¹³C NMR (126 MHz, DMSO-d₆) δ 167.0, 166.6, 140.5, 137.7, 134.8, 134.0, 132.1, 131.6, 129.5, 129.4, 129.1, 128.3, 128.1, 127.0, .9, 126.1; HRMS (FD-TOF) m/z: [M]+ C₂₂H₂₀N₂O₅S₂, calculated, 456.0808; found, 456.0734.

### 2.2. Synthesis of intermediates 25-KR-03 and 25-KR-27

The mixtures **4** (500 mg, 3.31 mmol) and **8** (1.28 g, 3.97 mmol), as well as DIPEA (1.15 mL, 6.62 mmol) were dissolved in 5.0 mL of ACN and stirred at 80 °C for 12 hours. The reaction mixture was diluted with EtOAc and washed with 1N aqueous HCl solution, saturated sodium bicarbonate and brine solution. The organic phase was dried over MgSO₄, filtered and concentrated. The residue was purified by flash column chromatography (n-hexane/EtOAc 7:3) to obtain light yellow oil **25-KR-03** (1.23 g, 3.14 mmol, 95% yield).

KOH (314 mg, 5.6 mmol) dissolved in methanol (6.0 mL) was added to **25-KR-03** (1.10 g, 2.80 mmol) and stirred at 35 °C until solids were formed. The mixture was diluted with water (30 mL) and then washed with Et₂O (30 mL). The pH of the aqueous layer was adjusted to 4 using 6N HCl aqueous solution. The aqueous mixture was extracted with Et₂O (30 mL × 2) and the organic phase was dried over MgSO₄, filtered and concentrated to obtain light brown solid **25-KR-27** (965 mg, 2.55 mmol, 91% yield), which was used for the next step without additional purification.

### Methyl 3-((2-oxo-2-((2-(phenylthio)phenyl)amino)ethyl)amino)benzoate (25-KR-03)

Light yellow oil (1.23 g), 95% yield; ¹H NMR (500 MHz, DMSO-d₆) δ 9.76 (s, 1H), 8.17 (d, *J =* 8.2Hz, 1H), 7.48 - 7.42 (m, 2H), 7.20 - 7.13 (m, 7H), 6.95 (dd, *J =* 7.9, 1.8Hz, 2H), 6.81 (dt, *J =* 7.2, 2.3Hz, 1H), 6.65 (t, *J =* 5.8, 1H), 3.84 (d, *J =* 5.9Hz, 2H), 3.79 (s, 3H); ¹³C NMR (126 MHz, DMSO-d₆) δ 169.3, 166.5, 148.1, 138.8, 135.4, 134.8, 130.3, 130.1, 129.2, 128.5, 126.7, 125.1, 123.2, 121.7, 118.0, 117.1, 112.8, 51.9, 47.999999999, 47.99999, 47.99999999999, 47.9; HRMS (FD-TOF) m/z: [M]+ C₂₂H₂₀N₂O_{3S}, calculated, 392.1189; found, 392.1189.

### 3-((2-oxo-2-((2-(phenylthio)phenyl)amino)ethyl)amino)benzoic acid (25-KR-27)

Light brown solid (965 mg), yield 91%; ¹H NMR (500 MHz, DMSO-d₆) δ 9.77 (s, 1H), 8.20 (d, *J* = 8.3Hz, 1H), 7.48 (dd, *J* = 7.7, 1.6Hz, 1H), 7.45 - 7.42 (m, 1H), 7.21 (dt, *J =* 7.6, 1.3Hz, 1H), 7.18 - 7.14 (m, 7H), 6.98 - 6.97 (m, 2H), 6.78 (ddd, *J* = 7.9, 2.6, 1.2Hz, 1H), 6.60 (t, *J=* 6.0Hz, 1H), 3.83 (d, *J=* 5.6Hz, 2H); ¹³C NMR (126 MHz, DMSO-d₆) δ 169.4, 167.6, 148.1, 138.9, 135.5, 134.7, 131.5, 130.1, 129.3, 129.1, 128.7, 126.8, 125.0, 123.1, 121.5, 118.3, 116.8, 113.1, 48.1, 48.1, 113.1; HRMS (FD-TOF) m/z: [M]+ C₂₁H₁₈N₂O₃S, calculated, 378.1033; found, 378.1035.

### 2.3. Ester synthesis from 25-KR-02 and 25-KR-27

*N,N'-* dicyclohexylcarbodiimide (**DCC**) (27 mg, 0.13 mmol) was dissolved in a mixture of **25-KR-02** or **25-KR-27** (0.10 mmol) and phenol (0.1 mmol) dissolved in CH₂Cl₂ (0.5 mL) at 25 °C, and stirred for 12 hours. After completion of the reaction, the reaction mixture was filtered through Celite and washed with EtOAc. The filtrate was washed with water and brine solution. The organic phase was dried over MgSO₄, filtered and concentrated in vacuo. The residue was purified by flash column chromatography (*n*-hexane/EtOAc 7:4) to produce ester derivatives of **25-KR-02** or **25-KR-27**.

### 4-(tert- butyl)phenyl 3-(N-(2-oxo-2-((2-(phenylthio)phenyl)amino)ethyl)methylsulfonamido)benzoate (25-KR-04)

Light yellow oil (47.1 mg), yield 80%; ¹H NMR (500 MHz, DMSO-d₆) δ 9.78 (s, 1H), 8.26 (t, *J=* 2.0Hz, 1H), 8.07 - 8.04 (m, 1H), 7.81 (ddd, *J =* 8.1, 2.3, 1.1Hz, 2H), 7.78 (d, *J* = 8.1Hz, 2H), 7.59 (t, *J* = 7.9Hz, 1H), 7.49 - 7.46 (m, 2H), 7.38 - 7.34 (m, 2H), 7.30 - 7.27 (m, 2H), 7.24 - 7.21(m, 2H), 7.20 - 7.16 (m, 5H), 4.60 (s, 2H), 3.18 (s, 3H), 1.31 (s, 9H); ¹³C NMR (126 MHz, DMSO-d₆) δ 167.0, 164.0, 148.4, 148.2, 140.7, 137.6, 134.8, 133.8, 132.6, 130.1, 129.9, 129.6, 129.4, 129.0, 128.7, 128.5, 127.0, 126.8, 126.3, 126.1, 124.3, 121.2, 53.8, 38.6, 34.2, 31.2; HRMS (FD-TOF) m/z: [M]+ C₃₂H₃₂N₂O₅S₂, calculated, 588.1747; found, 588.1749.

### o -Tolyl 3-(N -(2-oxo-2-((2-(phenylthio)phenyl)amino)ethyl)methylsulfonamido) benzoate (25-KR-05)

Light yellow oil (44.8 mg), yield 82%; ¹H NMR (400MHz, DMSO-d₆) δ 9.78 (s, 1H), 8.30 (t, *J* = 1.9Hz, 1H), 8.10 (d, *J* = 7.7Hz, 1H), 7.83 - 7.81 (m, 1H), 7.77 (d, *J* = 7.9Hz, 1H), 7.61 (t, *J* = 7.8Hz, 1H), 7.38 - 7.34 (m, 3H), 7.30 - 7.28 (m, 3H), 7.25 - 7.22 (m, 2H), 7.20 - 7.16 (m, 4H), 4.60 (s, 2H), 3.19 (s, 3H), 2.14 (s, 3H); ¹³C NMR (101 MHz, DMSO-d₆) δ 167.6, 164.1, 149.6, 141.4, 138.2, 135.3, 134.3, 133.3, 131.6, 130.6, 130.5, 130.4, 130.2, 130.0, 129.6, 129.2, 129.0, 127.7, 127.6, 127.4, 126.8, 126.7, 124.9, 122.7, 54.3, 39.2, 16.2; HRMS (FD-TOF) m/z: [M]+ C₂₉H₂₆N₂O₅S₂, calculated, 546.1278; found, 546.1276.

### m -Tolyl 3-(N-(2-oxo-2-((2-(phenylthio)phenyl)amino)ethyl)methylsulfonamido)benzoate (25-KR-06)

Light yellow oil (41.0 mg), yield 75%; ¹H NMR (400 MHz, DMSO-d₆) δ 9.78 (s, 1H), 8.27 (t, *J* = 1.9Hz, 1H), 8.05 (dt, *J* = 7.8, 1.3Hz, 1H), 7.82 - 7.77 (m, 2H), 7.60 (t, *J =* 7.9Hz, 1H), 7.38 - 7.27 (m, 5H), 7.25 - 7.22 (m, 1H), 7.21 - 7.16 (m, 3H), 7.13 (d, *J* = 7.4Hz, 1H), 7.06 - 7.04 (m, 2H), 4.60 (s, 2H), 3.18 (s, 3H), 2.34 (s, 3H); ¹³C NMR (101 MHz, DMSO-d₆) δ 167.6, 164.5, 151.0, 141.3, 139.9, 138.2, 135.3, 134.3, 133.3, 130.7, 130.5, 130.2, 130.0, 129.8, .6, 129.2, 129.0, 127.6, 127.3.3.3.3.3.3., 126.7, 124.9, 122.8, 119.4, 54.3, 39.2, 21.3; HRMS (FD-TOF) m/z: [M]+ C₂₉H₂₆N₂O₅S, calculated, 546.1278; found, 546.1261.

### p -Tolyl 3-(N-(2-oxo-2-((2-(phenylthio)phenyl)amino)ethyl)methylsulfonamido) benzoate (25-KR-07)

Light yellow oil (42.6 mg), yield 78%; ¹H NMR (400 MHz, DMSO-d₆) δ 9.55 (s, 1H), 8.26 (t, *J =* 2.0Hz, 1H), 8.06 - 8.04 (m, 1H), 7.83 - 7.79 (m, 2H), 7.61 - 7.56 (m, 1H), 7.39 - 7.34 (m, 2H), 7.30 - 7.22 (m, 5H), 7.21 - 7.18 (m, 3H), 7.16 - 7.12 (m, 2H), 4.59 (s, 2H), 3.17 (s, 3H), 2.35(s, 3H); ¹³C NMR (101 MHz, DMSO-D₆) δ 167.4, 164.5, 149.0, 141.5, 138.5, 135.8, 135.5, 134.5, 133.0, 131.0, 130.4, 130.2, 129.9, 129.6, 129.1, 128.9, 127.5, 127.3, 126.5., 124.7, 121.9, 54.5, 39.5, 20.9; HRMS (FD-TOF) m/z: [M]+ C₂₉H₂₆N₂O₅S₂, calculated, 546.1278; found, 546.1273.

### 2-Methoxyphenyl 3-(N-(2-oxo-2-((2-(phenylthio)phenyl)amino)ethyl)methylsulfonamido)benzoate (25-KR-08)

Light yellow oil (41.0 mg), yield 73%; ¹H NMR (400 MHz, DMSO-d₆) δ 9.78 (s, 1H), 8.26 (t, *J* = 2.0Hz, 1H), 8.05 (dt, *J* = 7.8, 1.3Hz, 1H), 7.81 - 7.77 (m, 2H), 7.59 (t, *J* = 7.9Hz, 1H), 7.38 - 7.34 (m, 2H), 7.31 - 7.27 (m, 2H), 7.25 - 7.22 (m, 1H), 7.21 - 7.17 (m, 5H), 7.02 - 6.98 (m, 2H), 4.59 (s, 2H), 3.78 (s, 3H), 3.18 (s, 3H); ¹³C NMR (101 MHz, DMSO-d₆) δ 167.6, 163.9, 151.5, 141.4, 139.8, 138.2, 135.4, 134.4, 133.2, 130.6, 130.4, 130.1, 130.0, 129.6, 129.3, 129.2, 127.8, 127.6, 127.3, 126.7, 125.0, 123.4, 121.2, 113.5, 56.3, 54.3, 39.2; HRMS (FD-TOF) m/z: [M]+ C₂₉H₂₆N₂O₆S₂, calculated, 562.1227; found, 562.1230.

### 4-Methoxyphenyl 3-(N-(2-oxo-2-((2-(phenylthio)phenyl)amino)ethyl)methylsulfonamido)benzoate (25-KR-09)

Light yellow oil (47.2 mg), yield 84%; ¹H NMR (400 MHz, DMSO-d₆) δ 9.78 (s, 1H), 8.26 (t, *J=* 2.0Hz, 1H), 8.05 (dt, *J* = 7.8, 1.3Hz, 1H), 7.81 - 7.77 (m, 2H), 7.59 (t, *J* = 7.9Hz, 1H), 7.38 - 7.34(m 2H), 7.31 - 7.27 (m, 2H), 7.25 - 7.22 (m, 1H), 7.21 - 7.17 (m, 5H), 7.02 - 6.98 (m, 2H), 4.59 (s, 2H), 3.78 (s, 3H), 3.18 (s, 3H); ¹³C NMR (101 MHz, DMSO-d₆) δ 167.6, 164.8, 157.6, 144.4, 141.3, 138.2, 135.4, 134.4, 133.2, 130.8, 130.5, 130.2, 130.0, 129.6, 129.2, 129.0, 127.6, 127.4, 126.7, 124.9, 123.2, 115.1, 56.0, 54.3, 39.2; HRMS (FD-TOF) m/z: [M]+ C₂₉H₂₆N₂O₆S₂, calculated, 562.1227; found, 562.1230.

### 4-Chlorophenyl 3-(N-(2-oxo-2-((2-(phenylthio)phenyl)amino)ethyl)methylsulfonamido)benzoate (25-KR-10)

Yellow solid (42.5 mg), yield 75%; ¹H NMR (400MHz, DMSO-d₆) δ 9.78 (s, 1H), 8.26 (t, *J* = 1.9Hz, 1H), 8.06 (d, *J* = 8.1Hz, 1H), 7.82 - 7.80 (m, 1H), 7.77 (d, *J* = 8.1Hz, 1H), 7.60 (t, *J* = 8.0Hz, 1H), 7.55 - 7.51 (m, 2H), 7.38 - 7.27 (m, 6H), 7.24 - 7.16 (m, 4H), 4.60 (s, 2H), 3.18 (s, 3H); ¹³C NMR (101 MHz, DMSO-D₆) δ 167.5, 164.3, 149.8, 141.3, 138.2, 135.4, 134.3, 133.4, 130.9, 130.5, 130.4, 130.2, 130.1, 130.0, 12.6, 129.3, 129.1, 127.6, 126.7, 124.9, 124.4, 54.3, 39.2; HRMS (FD-TOF) m/z: [M]+ C₂₈H₂₃ClN₂O₅S₂, calculated, 566.0731; found, 566.0744.

### 2-Cyanophenyl 3-(N-(2-oxo-2-((2-(phenylthio)phenyl)amino)ethyl)methylsulfonamido)benzoate (25-KR-11)

Yellow solid (33.4 mg), yield 60%; ¹H NMR (500 MHz, DMSO-d₆) δ 9.78 (s, 1H), 8.29 (t, *J=* 2.0Hz, 1H), 8.12 (d, *J* = 8.0Hz, 1H), 8.01 (dd, *J* = 7.8, 1.6Hz, 1H), 7.88 - 7.84 (m, 2H), 7.74 (d, *J* = 8.8Hz, 1H), 7.66 - 7.61 (m, 2H), 7.55 (t, *J* = 7.7Hz, 1H), 7.38 - 7.33 (m, 2H), 7.28 (t, *J* = 7.4Hz, 2H), 7.23 - 7.16 (m, 4H), 4.58 (s, 2H), 3.18 (s, 3H); ¹³C NMR (126 MHz, DMSO-d₆) δ 167.5, 163.6, 152.3, 141.5, 138.1, 135.6, 135.3, 134.2, 134.1, 133.8, 130.7, 130.1, 129.91, 129.88, 129.5, 129.34, 129.32, 129.1, 127.8, 129.1, 127.5, 126.7, 125.0, 124.1, 115.6, 106.7, 54.2, 39.2; HRMS (FD-TOF) m/z: [M]+ C₂₉H₂₃N₃O₅S₂, calculated, 557.1074; found, 557.1082.

### 3-Nitrophenyl 3-(N-(2-oxo-2-((2-(phenylthio)phenyl)amino)ethyl)methylsulfonamido)benzoate (25-KR-12)

Yellow solid (50.8 mg), 88% yield; ¹H NMR (500 MHz, DMSO-d₆) δ 9.80 (s, 1H), 8.29 (t, *J=* 2.0Hz, 1H), 8.23 (d, *J* = 1.9Hz, 1H), 8.20 (dt, *J* = 7.3, 1.9Hz, 1H), 8.09 (d, *J* = 8.0Hz, 1H), 7.83 - 7.75 (m, 4H), 7.62 (t, *J* = 8.0Hz, 1H), 7.38 - 7.27 (m, 4H), 7.24 - 7.16 (m, 4H), 4.60(s, 2H), 3.19 (s, 3H); ¹³C NMR (126 MHz, DMSO-d₆) δ 167.0, 163.6, 150.8, 148.3, 140.8, 137.6, 134.8, 133.7, 133.1, 130.9, 130.0, 129.7, 129.6, 129.4, 129.01, 128.98, 128.92, 128.6, 127.11., 127.0, 126.2, 124.4, 121.1, 117.5, 53.7, 38.7; HRMS (FD-TOF) m/z: [M]+ C₂₈H₂₃N₃O₇S₂, calculated, 577.0972; found, 577.0963.

### 4-Nitrophenyl 3-(N-(2-oxo-2-((2-(phenylthio)phenyl)amino)ethyl)methylsulfonamido)benzoate (25-KR-13)

Yellow solid (43.3 mg), yield 75%; ¹H NMR (500 MHz, DMSO-d₆) δ 9.79 (s, 1H), 8.37 - 8.33 (m, 2H), 8.28 (t, *J =* 2.0Hz, 1H), 8.08 (d, *J =* 8.0Hz, 1H), 7.83 (dd, *J =* 8.1, 1.3Hz, 1H), 7.75 (d, *J* = 7.8Hz, 1H), 7.63 - 7.58 (m, 3H), 7.38 - 7.27 (m, 4H), 7.24 - 7.16 (m, 4H), 4.60 (s, 2H), 3.18 (s, 3H); ¹³C NMR (126 MHz, DMSO-d₆) δ 167.0, 163.3, 155.4, 145.3, 140.8, 137.6, 134.8, 133.7, 133.1, 130.0, 129.7, 129.5, 129.4, 129.0, 128.9, 128.6, 127.1, 126.2, 125.3, 125.3, 124.4, 123.3, 53.7, 38.7; HRMS (FD-TOF) m/z: [M]+ C₂₈H₂₃N₃O₇S₂, calculated, 577.0972; found, 577.0964.

### Naphthalen-1-yl 3-(N-(2-oxo-2-((2-(phenylthio)phenyl)amino)ethyl)methylsulfonamido)benzoate (25-KR-14)

Yellow solid (36.7 mg), 63% yield; ¹H NMR (500 MHz, DMSO-d₆) δ 9.81 (s, 1H), 8.39 (t, *J =* 2.0Hz, 1H), 8.20 (d, *J =* 8.0Hz, 1H), 8.04 (d, *J* = 8.2Hz, 1H), 7.92 (d, *J =* 8.4Hz, 1H), 7.86 (d, *J* = 8.1Hz, 2H), 7.77 (d, *J* = 7.8Hz, 1H), 7.66 (t, *J* = 8.0Hz, 1H), 7.59 (t, *J* = 8.0Hz, 2H), 7.54 - 7.50 (m, 1H), 7.46 (d, *J* = 6.4Hz, 1H), 7.37 - 7.33 (m, 2H), 7.27 (t, *J* = 7.3Hz, 2H), 7.22 - 7.15(m, 4H), 4.62 (s, 2H), 3.21 (s, 3H); ¹³C NMR (126 MHz, DMSO-d₆) δ 167.0, 164.1, 146.2, 140.9, 137.6, 134.8, 134.2, 133.8, 133.0, 130.1, 129.8, 129.6, 129.4, 129.0, 128.9, 128.6, 128.1, 127.0, 126.9, 126.7, 126.3, 126.2, 125.8, 124.4, 120.9, 118.6, 53.8, 38.6; HRMS (FD-TOF) m/z: [M]+ C₃₂H₂₆N₂O₅S₂, calculated, 582.1278; found, 582.1268.

### Naphthalen-2-yl 3-(N-(2-oxo-2-((2-(phenylthio)phenyl)amino)ethyl)methylsulfonamido)benzoate (25-KR-15)

Light brown solid (39 mg), yield 67%; ¹H NMR (500MHz, DMSO-d₆) δ 9.80 (s, 1H), 8.31 (t, *J =* 2.0Hz, 1H), 8.11 (d, *J* = 7.8Hz, 1H), 8.03 (d, *J* = 8.9Hz, 1H), 8.00 (d, *J* = 7.4Hz, 1H), 7.94 (d, *J* = 7.3Hz, 1H), 7.83 - 7.81 (m, 2H), 7.76 (d, *J* = 7.5Hz, 1H), 7.62(t, *J* = 8.0Hz, 1H), 7.59 - 7.54 (m, 2H), 7.44 (dd, *J* = 8.8, 2.4Hz, 1H), 7.38 - 7.33 (m, 2H), 7.28 (t, *J* = 7.4Hz, 2H), 7.24 - 7.16 (m, 4H), 4.60 (s, 2H), 3.19 (s, 3H); ¹³C NMR (126 MHz, DMSO-d₆) δ 167.0, 164.1, 146.2, 140.9, 137.6, 134.8, 134.2, 133.8, 133.0, 130.1, 129.8, 129.6, 129.4, 129.0, 128.9, 128.6, 128.1, 127.0, 126.9, 126.7, 126.3, 126.21, 126.16, 125.8, 124.4, 120.9, 118.6, 53.8, 38.6; HRMS (FD-TOF) m/z: [M]+ C₃₂H₂₆N₂O₅S₂, calculated, 582.1278; found, 582.1274.

### 2-Methoxyphenyl 3-((2-oxo-2-((2-(phenylthio)phenyl)amino)ethyl)amino) benzoate (25-KR-20)

Yellow oil (36.3 mg), 75% yield; ¹H NMR (600 MHz, DMSO-d₆) δ 9.77 (s, 1H), 8.16 (d, *J=* 9.5Hz, 1H), 7.48 (dd, *J* = 7.7, 1.6Hz, 1H), 7.45 - 7.42 (m, 1H), 7.36 - 7.34 (m, 1H), 7.31 - 7.29 (m, 1H), 7.28 - 7.24 (m, 2H), 7.20 - 7.17 (m, 5H), 7.16 (t, *J =* 1.5Hz, 1H), 7.01 - 6.98 (m, 3H), 6.86 (ddd, *J* = 8.2, 2.5, 1.0Hz, 1H), 6.72 (t, *J* = 5.9Hz, 1H), 3.87 (d, *J* = 5.9Hz, 2H), 3.73 (s, 3H); ¹³C NM (151 MHz, DMSO-d₆) δ 169.3, 164.3, 151.0, 148.3, 139.4, 138.7, 135.4, 134.7, 130.1, 129.5, 129.4, 129.3, 128.5, 127.0, 126.7, 125.2, 123.4, 123.0, 121.9999, 120.6, 118.6, 117.4, 113.5, 112.9, 55.7, 47.8; HRMS (FD-TOF) m/z: [M]+ C₂₈H₂₄N₂O₄S, calculated, 484.1451; found, 484.1453.

### 4-Methoxyphenyl 3-((2-oxo-2-((2-(phenylthio)phenyl)amino)ethyl)amino) benzoate (25-KR-21)

Yellow oil (32.9 mg), 68% yield; ¹H NMR (600 MHz, DMSO-d₆) δ 9.77 (s, 1H), 8.15 (d, *J* = 8.2Hz, 1H), 7.47 - 7.42 (m, 2H), 7.36 - 7.34 (m, 1H), 7.28 - 7.24 (m, 2H), 7.20 - 7.12 (m, 6H), 6.99 - 6.98 (m, 4H), 6.89 - 6.87 (m, 1H), 6.71 (t, *J =* 5.9Hz, 1H), 3.87 (d, *J* = 6.1Hz, 2H), 3.77 (s, 3H); ¹³C NMR (151 MHz, DMSO-d₆) δ 169.3, 165.1, 156.9, 148.3, 144.0, 138.7, 135.3, 134.8, 130.0, 129.6, 129.5, 129.4, 129.3, 128.5, 125.2, 125.2, 125.7, 126.9, 118.5, 117.6, 114.5, 113.2, 55.4, 47.8; HRMS (FD-TOF) m/z: [M]+ C₂₈H₂₄N₂O₄S calculated, 484.1451; found, 484.1432.

### o-Tolyl 3-((2-oxo-2-((2-(phenylthio)phenyl)amino)ethyl)amino)benzoate (25-KR-22)

Light yellow oil (29.5 mg), yield 63%; ¹H NMR (600 MHz, DMSO-d₆) δ 9.78 (s, 1H), 8.15 (d, *J* = 8.2Hz, 1H), 7.47 - 7.45 (m, 1H), 7.44 - 7.42 (m, 1H), 7.40 - 7.39 (m, 1H), 7.33 - 7.32 (m, 2H), 7.29 - 7.26 (m, 2H), 7.22 - 7.19 (m, 1H), 7.18 - 7.14 (m, 5H), 6.99 - 6.98(m, 2H), 6.90 (ddd, *J =* 8.1, 2.5, 1.0Hz, 1H), 6.74 (t, *J =* 5.9Hz, 1H), 3.88 (d, *J =* 5.8Hz, 2H), 2.10 (s, 3H) ; ¹³C NMR (151 MHz, DMSO-d₆) δ 169.3, 164.5, 149.2, 148.4, 138.7, 135.3, 134.7, 131.0, 130.0, 129.8, 129.5, 129.4, 129.3, 128.6, 127.1, 126.7, 126.0, 125.2, 123.5, 123.5., 122.1, 121.9, 118.5, 117.8, 113.3, 47.8, 15.6; HRMS (FD-TOF) m/z: [M]+ C₂₈H₂₄N₂O₃S, calculated, 468.1502; found, 468.1514.

### m-Tolyl 3-((2-oxo-2-((2-(phenylthio)phenyl)amino)ethyl)amino)benzoate (25-KR-23)

Light yellow oil (27.2 mg), yield 58%; ¹H NMR (500 MHz, DMSO-d₆) δ 9.77 (s, 1H), 8.15 (d, *J* = 8.2Hz, 1H), 7.47 - 7.42 (m, 2H), 7.36 - 7.34 (m, 1H), 7.29 - 7.24 (m, 4H), 7.20 - 7.15 (m, 4H), 7.10 - 7.08 (m, 2H), 6.99 - 6.97 (m, 2H), 6.88 (ddd, *J =* 8.1, 2.6, 1.1Hz, 1H), 6.71 (t, *J* = 5.8Hz, 1H), 3.88 (d, *J* = 5.8Hz, 2H), 2.33 (s, 3H); ¹³C NMR (126 MHz, DMSO-d₆) δ 169.8, 165.4, 151.1, 148.8, 139.8, 139.2, 135.7, 135.2, 130.5, 130.1, 129.9, 129.8, 129.7, 129.0, 127.2, 127.0, 125.7, 124.0, 122.7., 122.4, 119.3, 119.0, 118.2, 113.7, 48.3, 21.3; HRMS (FD-TOF) m/z: [M]+ C₂₈H₂₄N₂O₃S, calculated, 468.1502; found, 468.1510.

### p-Tolyl 3-((2-oxo-2-((2-(phenylthio)phenyl)amino)ethyl)amino)benzoate (25-KR-24)

Light yellow oil (32.3 mg), yield 69%; ¹H NMR (500 MHz, DMSO-d₆) δ 9.78 (s, 1H), 8.15 (d, *J* = 8.2Hz, 1H), 7.47 - 7.42 (m, 2H), 7.37 - 7.34 (m, 1H), 7.33 - 7.31 (m, 1H), 7.29 - 7.25 (m, 2H), 7.21 - 7.15 (m, 5H), 7.12 - 7.10 (m, 1H), 7.03 - 7.01 (m, 1H), 7.00 - 6.98 (m, 2H), 6.89 (ddd, *J* = 8.1, 2.6, 1.1Hz, 1H), 6.72 (t, *J* = 5.9Hz, 1H), 3.88 (d, *J =* 5.9Hz, 2H), 2.34 (s, 3H); ¹³C NMR (126 MHz, DMSO-d₆) δ 169.3, 165.0, 148.4, 148.3, 138.7, 135.3, 135.0, 134.8, 130.0, 129.9, 129.6, 129.4, 129.3, 128.5, 126.7, 125.2, 123.5, 121.9, 121.5., 118.5, 117.6, 113.3, 47.8, 20.4; HRMS (FD-TOF) m/z: [M]+ C₂₈H₂₄N₂O₃S, calculated, 468.1502; found, 468.1489.

### 4-Chlorophenyl 3-((2-oxo-2-((2-(phenylthio)phenyl)amino)ethyl)amino) benzoate (25-KR-25)

Yellow solid (36.1 mg), 74% yield; ¹H NMR (600 MHz, DMSO-d₆) δ 9.77 (s, 1H), 8.14 (d, *J* = 8.2Hz, 1H), 7.53 - 7.50 (m, 2H), 7.46 - 7.42 (m, 2H), 7.37 - 7.35 (m, 1H), 7.30 - 7.25 (m, 4H), 7.20 - 7.14 (m, 4H), 6.98 (d, *J* = 6.7Hz, 2H), 6.90 (ddd, *J* = 8.2, 2.6, 1.0Hz, 1H), 6.73 (t, *J* = 5.9Hz, 1H), 3.88 (d, *J* = 6.0Hz, 2H); ¹³C NMR (151 MHz, DMSO-d₆) δ 169.2, 164.7, 149.4, 148.3, 138.7, 135.2, 134.8, 130.04, 130.00, 129.5, 129.3, 129.2, 128.5, 126.7, 1 25.2, 123.9, 123.5, 122.0, 118.6, 117.9, 113.3, 47.7; HRMS (FD-TOF) m/z: [M]+ C₂₇H₂₁ClN₂O₃S, calculated, 488.0956; found, 488.0936.

### 2.4. Synthesis of intermediate 25-KR-26

A mixture of **25-KR-03** (39.2 mg, 0.1 mmol), *p-toluene* sulfonyl chloride (9) (22.9 mg, 0.12 mmol) and 4-dimethylaminopyridine (DMAP) (24.4 mg, 0.2 mmol) was added and dissolved in CH₂Cl₂ (0.5 mL), and then stirred at 25 °C for 12 hours. The reaction mixture was diluted with EtOAc and washed with 1N aqueous HCl solution, saturated sodium bicarbonate and brine solution. The organic phase was dried over MgSO₄, filtered and concentrated. The residue was purified by flash column chromatography (n-hexane/EtOAc 7:3) to obtain yellow solid **25-KR-26** (32.8 mg, 0.06 mmol, 60% yield).

### Methyl 3-((4-methyl-N-(2-oxo-2-((2-(phenylthio)phenyl)amino)ethyl)phenyl)sulfonamido)benzoate (25-KR-26)

Yellow solid (32.8 mg), yield 60%; ¹H NMR (600MHz, DMSO-d₆) δ 9.68 (s, 1H), 7.83 - 7.80 (m, 2H), 7.70 (d, *J =* 8.1Hz, 1H), 7.50 (d, *J =* 8.3Hz, 2H), 7.43 - 7.38 (m, 3H), 7.36 - 7.32 (m, 3H), 7.30 - 7.28 (m, 2H), 7.24 - 7.21 (m, 1H), 7.17 - 7.14 (m, 3H), 4.46 (s, 2H), 3.81 (s, 3H), 2.39 (s, 3H); ¹³C NMR (151 MHz, DMSO-D₆) δ 166.0, 165.4, 144.1, 139.9, 137.7, 134.8, 134.4, 133.8, 132.0, 130.3, 129.8, 129.48, 129.45, 129.4, 129.0, 128.7, 128.3, 127.5, 127.0.0.0.0.0.0, 126.4, 126.0, 124.1, 53.4, 52.3, 21.0; HRMS (FD-TOF) m/z: [M]+ C₂₉H₂₆N₂O₅S₂, calculated, 546.1278; found, 546.1287.

### 2.5. Synthesis of intermediates 25-KR-16 and 25-KR-17

3-Nitrophenol (**10**) (1.00 g, 7.19 mmol), *tert*-butyldimethylsilyl chloride (TBDMSCl) (1.63 g, 10.79 mmol) and imidazole (978.90 mg, 14.38 mmol) were dissolved in CH₂Cl₂ (20 mL) and stirred at 25 °C for 6 hours. The mixture was diluted with CH₂Cl₂ and washed with water and brine solution (NaCl). The organic phase was dried over MgSO₄, filtered and concentrated. The residue was purified by flash column chromatography (*n*-hexane/EtOAc 8:2) to obtain light yellow oil **11** (1.79 g, 7.05 mmol, 98% yield).

Zinc (1.38 g, 21.15 mmol) and acetic acid (4.03 mL, 70.50 mmol) were added dropwise to a mixture of **11** (1.79 g, 7.05 mmol) dissolved in ethanol and water (v/v 9: 1) (18 mL). The mixture was stirred at 25 °C for 4 hours. The reaction mixture was filtered through Celite and washed with excess ethanol. The filtrate was evaporated to dryness to obtain a residue, then EtOAc (50 mL) was added thereto, followed by washing with saturated sodium bicarbonate and brine solution. The organic phase was dried over MgSO₄, filtered and concentrated. The crude material was purified by flash column chromatography (n-hexane/EtOAc 7:3) to obtain light yellow oil **12** (1.49 g, 6.70 mmol, 95% yield).

Pyridine (0.50 mL, 6.26 mmol) was added to a mixture of **12** (700 mg, 3.13 mmol) dissolved in CH₂Cl₂ (21 mL) and cooled to 0 °C. Methanesulfonyl chloride (0.29 mL, 3.76 mmol) was added to the mixture and stirred at 25 °C for 12 hours. The reaction mixture was washed with 1N aqueous HCl solution, saturated sodium bicarbonate and brine solution. The organic phase was dried over MgSO₄, filtered and concentrated to obtain the crude material. Then, solvent (7 mL, n-hexane/EtOAc 9:1) was added thereto, and the mixture was stirred for 10 minutes, then filtered and dried to yield pale yellow solid **13** (850.2 mg, 2.82 mmol, 90% yield).

ACN (8.4 mL) was added to a mixture of **13** (850.20 mg, 2.82 mmol), **8** (1.09 g, 3.38 mmol) and DIPEA (0.98 mL, 5.64 mmol) and stirred at 80 °C for 12 hours. The reaction mixture was diluted with EtOAc and washed with 1N aqueous HCl solution, saturated sodium bicarbonate and brine solution. The organic phase was dried over MgSO₄, filtered and concentrated. The residue was purified by flash column chromatography (*n*-hexane/EtOAc 7:3) to obtain white solid **25-KR-16** (1.41 g, 2.59 mmol, 92% yield).

1.0M TBAF in THF (0.2 mL, 0.20 mmol) was added dropwise to **25-KR-16** (54.30 mg, 0.10 mmol) dissolved in furan (THF) (2.5 mL) and stirred at 25 °C for 2 hours. After evaporating THF under reduced pressure, the mixture was diluted with EtOAc and washed with water and brine solution. The organic phase was dried over MgSO₄, filtered and concentrated in vacuo. The residue was purified by flash column chromatography (*n*-hexane/EtOAc 6:4) to obtain white solid **25-KR-17** (40.7 mg, 0.095 mmol, 95% yield).

### 2-(N-(3-((tert-butyl dimethylsilyl)oxy)phenyl)methylsulfonamido)-N-(2-(phenylthio)phenyl)acetamide (25-KR-16)

White solid (1.41 g), 92% yield; ¹H NMR (500 MHz, DMSO-d₆) δ 9.70 (s, 1H), 7.83 (d, *J =* 7.5Hz, 1H), 7.41 - 7.36 (m, 2H), 7.33 - 7.29 (m, 2H), 7.26 - 7.17 (m, 5H), 7.04 - 7.02 (m, 2H), 6.80 (ddd, *J* = 8.2, 2.3, 1.0Hz, 1H), 4.47 (s, 2H), 3.10 (s, 3H), 0.92 (s, 9H), 0.16 (s, 6H); ¹³C NMR (126 MHz, DMSO-d₆) δ 167.1, 155.5, 141.2, 137.8, 134.1, 130.0, 129.8, 129.6, 129.4, 129.2, 127.1, 125.9, 123.6, 120.0, 119.0, 118.8, 54.1, 38.1, 25.5.5., 17.9, -4.6; HRMS (FD-TOF) m/z: [M]+ C₂₇H₃₄N₂O₄S₂Si, calculated, 542.1724; found, 542.1717.

### 2-(N-(3-hydroxyphenyl)methylsulfonamido)-N-(2-(phenylthio)phenyl)acetamide (25-KR-17)

White solid (40.7 mg), 95% yield; ¹H NMR (500 MHz, DMSO-d₆) δ 9.68 (d, *J* = 3.3Hz, 2H), 7.87 (d, *J* = 8.2Hz, 1H), 7.41 - 7.37 (m, 2H), 7.33 - 7.29 (m, 2H), 7.25 - 7.22 (m, 1H), 7.21 - 7.17 (m, 3H), 7.12 (t, *J* = 8.0Hz, 1H), 6.92 (t, *J* = 2.3Hz, 1H), 6.82 (ddd, *J* = 8.0, 2.1, 1.0Hz, 1H), 6.71 (ddd, *J =* 8.2, 2.4, 1.0Hz, 1H), 4.43 (s, 2H), 3.10 (s, 3H); ¹³C NMR (126 MHz, DMSO-D₆) δ 167.2, 157.9, 141.2, 138.0, 134.8, 134.3, 129.8, 129.44, 129.39, 129.3, 127.0, 125.9, 125.7, 123.6, 117.5, 114.7, 114.4, 54.2, 38.2, 38.2; HRMS (FD-TOF) m/z: [M]+ C₂₁H₂₀N₂O₄S₂, calculated, 428.0859; found, 428.0857.

### 2.6. Synthesis of intermediates 25-KR-18 and 25-KR-19

A mixture of **12** (201.1 mg, 0.90 mmol), **8** (348 mg, 1.08 mmol) and DIPEA (0.31 mL, 1.80 mmol) was dissolved in ACN (2.0 mL) and stirred at 80 °C for 12 hours. The reaction mixture was diluted with EtOAc and washed with 1N aqueous HCl solution, saturated sodium bicarbonate and brine solution. The organic phase was dried over MgSO₄, filtered and concentrated. The residue was purified by flash column chromatography (*n*-hexane/EtOAc 7:3) to obtain white solid **25-KR-18** (372.2 mg, 0.80 mmol, 89% yield).

1.0M TBAF (0.2 mL, 0.2 mmol) in THF was added dropwise to **25-KR-18** (46.5 mg, 0.1 mmol) dissolved in THF (2.5 mL) and stirred at 25 °C for 2 hours. After evaporating THF under reduced pressure, the mixture was diluted with EtOAc and washed with water and NaCl. The organic phase was dried over MgSO₄, filtered and concentrated in vacuo. The residue was purified by flash column chromatography (n-hexane/EtOAc 6:4) to obtain white solid **25-KR-19** (34 mg, 0.097 mmol, 97% yield).

### 2-((3-((tert-butyldimethylsilyl)oxy)phenyl)amino)-N-(2-(phenylthio)phenyl)acetamide (25-KR-18)

White solid (372.2 mg), yield 89%; ¹H NMR (500 MHz, DMSO-d₆) δ 9.79 (s, 1H), 8.24 (d, *J =* 6.9Hz, 1H), 7.49 (dd, *J* = 7.7, 1.5Hz, 1H), 7.44 - 7.40 (m, 1H), 7.23 - 7.18 (m, 3H), 7.13(td, *J* = 7.6, 1.5Hz, 1H), 7.07 - 7.05 (m, 2H), 6.94 (t, *J* = 8.0Hz, 1H), 6.39 (t, *J =* 5.7Hz, 1H), 6.21 (ddd, *J* = 8.2, 2.3, 0.9Hz, 1H), 6.13 (ddd, *J =* 7.9, 2.3, 0.9Hz, 1H), 6.08 (t, *J* = 2.2Hz, 1H), 3.75 (d, *J* = 5.7Hz, 2H), 0.88 (s, 9H), 0.10 (s, 6H); ¹³C NMR (126 MHz, DMSO-D₆) δ 169.6, 156.0, 149.3, 138.8, 135.4, 134.7, 130.1, 129.8, 129.3, 129.2, 127.0, 124.8, 123.0, 121.1, 109.0, 106.3, 104.2, 48.3, 25., 17.9, -4.5; HRMS (FD-TOF) m/z: [M] + C₂₆H₃₂N₂O₂SSi, calculated, 464.1948; found, 464.1948.

### 2-((3-Hydroxyphenyl)amino)-N-(2-(phenylthio)phenyl)acetamide (25-KR-19)

White solid (34 mg), 97% yield; ¹H NMR (500 MHz, DMSO-d₆) δ 9.83 (s, 1H), 9.09 (s, 1H), 8.26 (d, *J* = 8.2Hz, 1H), 7.52 (dd, *J* = 7.8, 1.6Hz, 1H), 7.45 - 7.41 (m, 1H), 7.23 - 7.18 (m, 3H), 7.14(td, *J* = 7.6, 1.4Hz, 1H), 7.07 - 7.05 (m, 2H), 6.86 (t, *J* = 7.9Hz, 1H), 6.26 (t, *J* = 5.8Hz, 1H), 6.09 - 6.08 (m, 1H), 6.05 - 6.02 (m, 2H), 3.71 (d, *J* = 5.6Hz, 2H); ¹³C NMR (126 MHz, DMSO-d₆) δ 169.8, 158.3, 149.3, 138.9, 135.7, 134.6, 130.2, 129.7, 129.4, 129.3, 127.1, 124.8, 122.9, 120.9, 109.0, 109.1; HRMS (FD-TOF) m/z: [M]+ C₂₀H₁₈N₂O₂S, calculated, 350.1084; found, 350.1077.

### 3. Biological experiment method

### 3.1. Materials and reagents

PMSA-3-Ac was purchased from ChemBridge and dissolved in DMSO (Sigma-Aldrich, St. Louis, MO, USA) as previously described. Anti-β (#A5441) was purchased from Sigma-Aldrich. Anti-cathepsin K (#48353), anti-TRAF6 (B-10) and anti-c-Src (#sc-8056) were obtained from Santa Cruz Biotechnology, Inc. (Dallas, TX, USA). Anti-ubiquitin, lys48-specific, anti-c-fos (#4384s), anti-phospho-NF-κ (#3033s), anti-NF-κ and anti-NFATc1 (#8032s) were obtained from Cell Signaling Technology (Boston, Massachusetts, USA). Horseradish peroxidase-conjugated secondary antibody (#7074S) was provided by Cell Signaling Technology. The ECL system (#RPN2106) for chemiluminescent signal detection was purchased from iNtRON (Seoul, Korea).

### 3.2. Isolation and culture of bone marrow-derived macrophages

Bone marrow cells (BMC) were obtained from 8-week-old female C57/BL6 mice by washing femurs and tibias with α-MEM. BMCs were then cultured overnight in full α-MEM containing 10% FBS, 100 U/mL penicillin, and 100 µg/mL streptomycin. Non-attached cells were collected and cultured with M-CSF (30 ng/ mL) for 3 days, and then the attached cells were used as BMM. All animal experiments were approved by Chonnam National University IACUC (approval number: CNU IACUC-YB-2019-49).

### 3.3. In vitro osteoclast formation and cell viability assay

To generate osteoclasts, BMMs were harvested and cultured in a complete medium containing 30 ng/mL M-CSF and 50 ng/mL RANKL with or without compounds for approximately 4 days to obtain mature osteoclasts. Differentiated osteoclasts were fixed with 4% formalin for 15 minutes, and then the cells were stained using a TRAP staining kit. The spread of osteoclast areas could be observed under the microscope. The viability of BMM (1 × 10⁴ cells/well) was assessed in a 96-well plate using a cell viability assay kit as previously described.

### 3.4. Bone resorption and F-actin ring immunofluorescence analyses

Bone resorption analysis was performed by a conventional method. Briefly, BMMs (2 × 10⁴ cells/well) were seeded into a coated 48-well plate provided in the kit. After culturing with or without the indicated doses of 25-KR-03 and PMSA-3-Ac until mature osteoclasts were observed, 100 µL supernatant was harvested from each well and placed in a black polypropylene 96-well microplate. Fluorescence intensity was measured using a SpectraMax i3x fluorescence plate reader (excitation wavelength 485 nm; emission wavelength 535 nm). The decalcified pit area was visible under a microscope and calculated using ImageJ software. F-actin rings were visible using a rhodamine-coupled phalloidin staining assay (Thermo Fisher Scientific) as previously described.

### 3.5. RNA isolation and quantitative real-time PCR

Total RNA from BMM was isolated using QIAzol RNA lysis reagent (Qiagen Sciences, Valencia, CA, USA). Detailed information on primers used for real-time PCR analysis is listed in Table 1.

**[TABLE 1]**

| Gene | Primer sequence (5' → 3') | |
|---|---|---|
| *Cathepsin K* | Forward | GGACGCAGCGATGCTAACTAA (SEQ ID NO: 1) |
| | Reverse | CAGAGAGAAGGGAA (SEQ ID NO: 2) |
| *Acp5* | Forward | CAGCTGTCCTGGCTCAAAA (SEQ ID NO: 3) |
| | Reverse | ACATAGCCCACACCGTTCTC (SEQ ID NO: 4) |
| *DC-STAMP* | Forward | CGCACGATGCTTCATTCTTC (SEQ ID NO: 5) |
| | Reverse | CAGTGCCAGCCGCAATC (SEQ ID NO: 6) |
| *GAPDH* | Forward | TGTGTCCGTCGTGGATCTGA (SEQ ID NO: 7) |
| | Reverse | GATGCCTGCTTCACCACCTT (SEQ ID NO: 8) |
| *MMP9* | Forward | CTGGACAGCCAGACACTAAAG (SEQ ID NO: 9) |
| | Reverse | CTCGCGGCAAGTCTTCAGAG (SEQ ID NO: 10) |
| *NFATc1* | Forward | ACCACCTTTCCGCAACCA (SEQ ID NO: 11) |
| | Reverse | GGTACTGGCTTCTCTTCCGTTTC (SEQ ID NO:12) |
| *ATP6v0d2* | Forward | ACTATGGCCACCCGGGAAT (SEQ ID NO: 13) |
| | Reverse | GGCCCAAGGGAGTCATGTG (SEQ ID NO: 14) |

### 3.6. Western blotting analysis

Total protein was extracted from osteoclasts and processed by a conventional method. To obtain total cell lysates, cells were lysed in ice-cold RIPA buffer for 30 min. After measuring the total protein concentration using the Enhanced BCA Protein Assay Kit, the protein was separated using 8-12% SDS-PAGE and transferred to a polyvinylidene fluoride membrane. Next, the membrane was blocked using 5% skim milk and incubated with a primary antibody (1:2,000 dilution) overnight. After incubation with a secondary antibody (1:2,000 dilution) for 1 hour, the membrane was washed and developed using an enhanced chemiluminescence (ECL) buffer as previously described. Relative protein expression was calculated by densitometric analysis using Image J software.

### 3.7. OVX-induced osteoporosis mouse model

Specific pathogen-free facilities were used to grow mice. To investigate the effects of 25-KR-03 *in vivo*, the inventors established an OVX-induced osteoporosis mouse model as previously described. Seven-week-old C57BL/6 female mice were divided into four groups (Sham, OVX, 25-KR-03, and PMSA-3-Ac). Among them, the OVX, 25-KR-03, and PMSA-3-Ac groups underwent OVX surgery. The sham group had an abdominal incision.

After a 1-week recovery period, mice were intraperitoneally injected with 25-KR-03 or PMSA-3-Ac (20 mg/kg, dissolved in a mixture of 10 % Tween 80 and 10% DMSO) once daily for 4 weeks. The sham and control (OVX) groups received equal volumes of the mixture of 10 % Tween 80 and 10% DMSO once daily for 4 weeks. Body weights of the mice were measured weekly. When the mice were euthanized, the femurs were isolated and fixed in 4.0% paraformaldehyde for micro-computed tomography (micro-CT) analysis as previously performed.

### 3.8. Micro CT scanning

Micro-CT scanning was performed as in previous studies. The Quantum GX Micro-CT imaging system (PerkinElmer, Hopkinton, MA, USA) was used at the Korea Basic Science Institute (Gwangju, Korea).

### 3.9. Statistical analysis

Differences between multiple groups were compared using one-way analysis of variance (ANOVA) with Dunnett's multiple comparison test (GraphPad Prism 8.3.0, San Diego, CA, USA). Unpaired Student's t-test was used to compare differences between the two groups (^{*} *p* <0.05; *^{**} p* <0.01; *^{***} p* <0.001; NS, not significant). All data are expressed as mean ± SD.

### Experiment Result

### 1. Chemical experiment results

### 1.1. Synthesis of 25-KR-# derivatives

Based on the activity of PMSA-3-Ac, a PMSA-3 type moiety was designed to discover potent candidates. New compounds were designed with a core skeleton of PMSA substituted at the third position of the phenyl ring, especially with ester and alcohol derivatives.

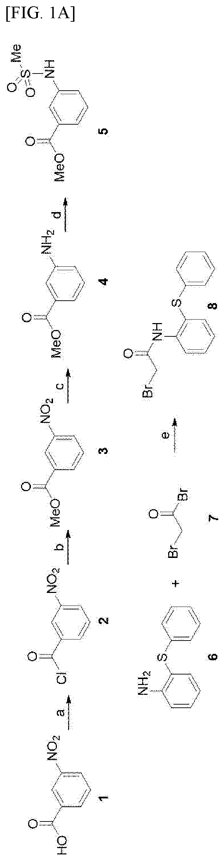

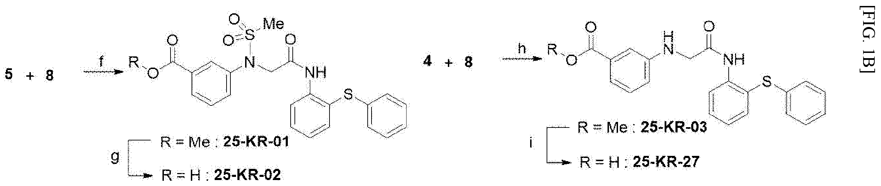

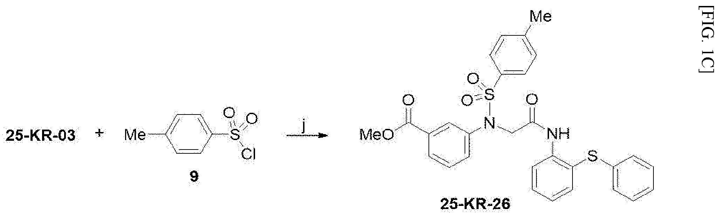

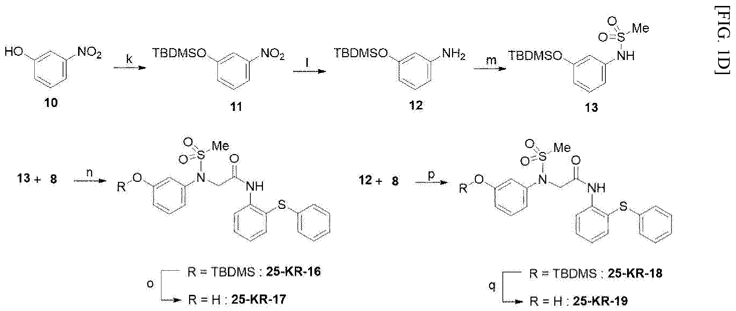

To synthesize desired compounds through late functionalization, intermediates 25-KR-02 and 25-KR-27 were synthesized as shown in FIG. 1.

The chemical structures of PMSA-3-Ac and 25-KR-# derivatives are shown in Formulas 2 and 3 below, respectively.

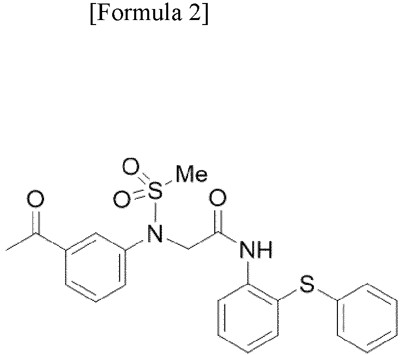

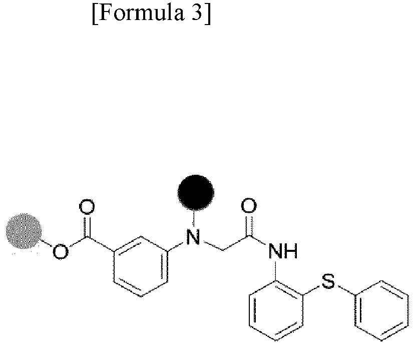

In FIG. 1, the synthetic route of the 25-KR-# derivative is schematically illustrated. Reaction conditions for each step are as follows: (a) (COCl)₂, dimethylformamide (DMF), CH₂Cl₂, 0 °C to rt, 4 hours; (b) Et₃N, MeOH, CH₂Cl₂, room temperature, 5 hours; (c) Zn, AcOH, EtOH:H2O, room temperature, 4 hours; (d) mesyl chloride, pyridine, CH₂Cl₂, 0 °C to rt, 12 hours; (e) Et₃N, CH₂Cl₂, 0 °C to rt, 8 hours; (f) diisopropylethyl amine (DIPEA), acetonitrile (ACN), 80 °C, 12 hours; (g) KOH, 40 °C, 2 hours, H₂O, 6N HCl (pH = 4); (h) DIPEA, ACN, 80 °C, 12 hours; (i) KOH, 40 °C, 2 hours, H₂O, 6N HCl (pH = 4); (j) 4-dimethylaminopyridine (DMAP), CH₂Cl₂, room temperature, 12 hours; (k) *tert-*butyldimethyl silyl chloride (TBDMSCl), imidazole, CH₂Cl₂, rt, 6 hours; (l) Zn, AcOH, EtOH:H₂O, room temperature, 4 hours; (m) mesyl chloride, pyridine, CH₂Cl₂, 0 °C to rt, 12 hours; (n) DIPEA, ACN, 80 °C, 12 hours; (o) tetrabutylammonium fluoride (TBAF), tetrahydrofuran (THF), room temperature, 2 hours; (p) DIPEA, ACN, 80 °C, 12 hours; (q) TBAF, THF, RT, 2 hours.

Specifically, 3-nitrobenzoic acid (**1**) was reacted with oxalyl chloride to produce the corresponding acid chloride (**2**). The acid chloride was converted into the methyl ester (**3**) by treatment with methanol in the presence of a base. Methyl-3-nitrobenzoate (**3**) was reduced to the corresponding aminobenzoate (**4**). Mesylation of the amine group of 4 produced intermediate 5. In a separation reaction, 2-(phenylthio)aniline (**6**) was reacted with bromoacetyl bromide (**7**) in the presence of a base to produce the corresponding acetamide (**8**).

**25-KR-01** was produced through a substitution reaction between compounds **5** and **8** in the presence of diisopropylethyl amine (DIPEA). Subsequent hydrolysis of the resulting product produced the corresponding acid, **25-KR-02,** in excellent yield. Likewise; the reaction of compound **4** with compound **8** in the presence of DIPEA yielded **25-KR-03**, which was hydrolyzed to give compound **25-KR-27**. Further, the reaction between **25-KR-03** and 4-methylbenzenesulfonyl chloride (**9**) yielded the *N*-tosylation product **25-KR-26**.

Compound **11** was obtained by protecting the hydroxyl group of 3-nitrophenol (**10**) with a silyl group in the presence of imidazole. The nitro group of **11** was reduced to the amino group using zinc and acetic acid to obtain **12**, which was then mesylated to produce intermediate **13**. Compounds **13** and **8** were reacted in the presence of a base to obtain **25-KR-16**, which was silyl deprotected with TBAF to obtain **25-KR-17** in excellent yield. Compound **12** was reacted with compound **8** in the presence of a base to produce compound **25-KR-18**, which was also silyl-deprotected with TBAF to yield **25-KR-18** in excellent yield. Applying terminal modifications to synthesize ester derivatives from **25-KR-02** using *N,N'* -dicyclohexylcarbodiimide (DCC) and 4-(dimethylamino)pyridine (DMAP), the corresponding compounds **25-KR-04** to **25-KR-15** were produced. Additionally, ester derivatives **25-KR-20** to **25-KR-25** were synthesized from **25-KR-27.**

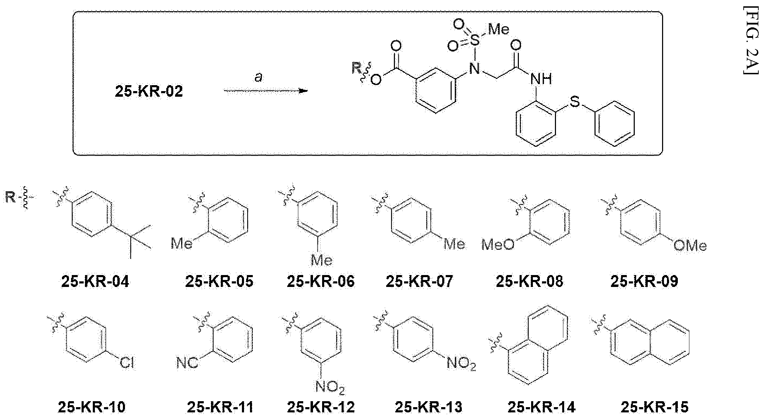

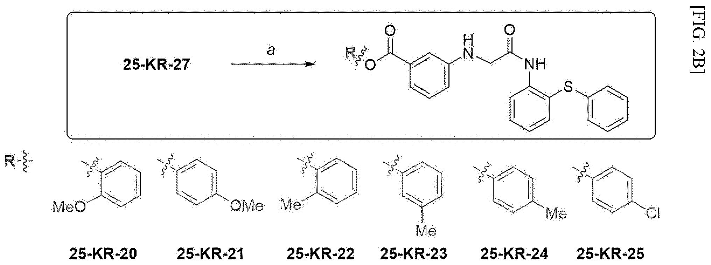

In FIG. 2, the synthetic route of the 25-KR-# derivative is schematically illustrated. Corresponding reaction conditions are as follows: (a) N,N'-dicyclohexylcarbodiimide DCC, CH₂Cl₂; rt, 12 hours.

### 2. Biological experiment results

### 2.1. Screening of PMSA-3 type moiety derivatives

An increase in the number and activity of mature osteoclasts is a hallmark of osteoclast-mediated bone loss. To discover more potent compounds to prevent osteoclast formation, 19 novel PMSA-3-Ac derivatives (25-KR-01 to 19) were synthesized and analyzed using TRAP staining to confirm the formation of mature osteoclasts.

Among all the novelsynthetic PMSA-3-Ac derivatives, 25-KR-03 showed the strongest inhibitory effect on osteoclast differentiation, and further showed almost complete inhibition at 2 µM. Next, a novel derivative without sulfur dioxide was synthesized as shown in Scheme 2 (25-KR-20~27), and TRAP staining was performed again to analyze its bioactivity in inhibiting osteoclast formation. Surprisingly, among all the novel synthetic PMSA-3-Ac derivatives, 25-KR-03 still showed the strongest inhibitory effect on osteoclast differentiation.

The structure-activity relationship (SAR) analysis results in Tables 2 and 3 below show that 25-KR-03, a PMSA-3 derivative without a mesityl substituent on the amino group, exhibits a greater inhibitory effect than other PMSA-3 derivatives.

**[TABLE 2]**

| Osteoclast formation inhibitory effect of novel synthetic PMSA-3-Ac derivative (25-KR-01 to 19) (%) | | | |
|---|---|---|---|
| Compound | Inhibition rate (%)^{a,b} | Compound | **Inhibition** rate (%)^{a,b} |
| 25-KR-01 | Inactive ^{c} | 25-KR-11 | Inactive ^{c} |
| 25-KR-02 | Inactive ^{c} | 25-KR-12 | Inactive ^{c} |
| 25-KR-03 | 99.89 ± 0.04 | 25-KR-13 | Inactive ^{c} |
| 25-KR-04 | Inactive ^{c} | 25-KR-14 | Inactive ^{c} |
| 25-KR-05 | Inactive ^{c} | 25-KR-15 | Inactive ^{c} |
| 25-KR-06 | Inactive ^{c} | 25-KR-16 | Inactive ^{c} |
| 25-KR-07 | Inactive ^{c} | 25-KR-17 | Inactive ^{c} |
| 25-KR-08 | 37.58 ± 8.21 | 25-KR-18 | Inactive ^{c} |
| 25-KR-09 | 45.80 ± 3.65 | 25-KR- 19 | Inactive ^{c} |
| 25-KR-10 | 47.37 ± 9.79 | Alendronate | 41.16 ± 8.68 |

| | | | |
|---|---|---|---|
| a: The concentration (%) of inhibiting osteoclast differentiation in RANKL-induced BMM in the table is 2 µM. Inhibition rate (%) is expressed as mean ± SD from at least three experiments. b: All active compounds were tested at non-cytotoxic concentrations. c: "Inactive" indicates that the % inhibition is less than 30%. | | | |

**[TABLE 3]**

| Osteoclast formation inhibitory effect of novel synthetic PMSA-3-Ac derivative (25-KR-20 to 27) (%) | | | |
|---|---|---|---|
| Compound | Inhibition rate (%)^{a,b} | Compound | Inhibition rate (%)^{a,b} |
| 25-KR-03 | 72.27 ± 6.33 | 25-KR-24 | 36.82 ± 7.82 |
| 25-KR-20 | 52.27±1.11 | 25-KR-25 | 43.18 ± 2.32 |
| 25-KR-21 | 50.91 ± 1.93 | 25-KR-26 | 49.09 ± 2.32 |
| 25-KR-22 | 47.73±11.43 | 25-KR-27 | 31.82 ± 1.93 |
| 25-KR-23 | 46.82 ± 13.36 | - | - |

| | | | |
|---|---|---|---|
| a: The concentration (%) of inhibiting osteoclast differentiation in RANKL-induced BMM in the table is 1 µM. Inhibition rate (%) is expressed as mean ± SD from at least three experiments. b: All active compounds were tested at non-cytotoxic concentrations. | | | |

Further, 25-KR-03 has a methyl ester group on the N-phenyl substituent, whereas other PMSA-3 derivatives have an aryl ester group on the N-phenyl substituent. Further, among PMSA-3 derivatives with an aryl ester group on the N-phenyl substituent, phenyl rings of 25-KR-08, 25-KR-09 and 25-KR-10 esters with ortho-methoxy, para-methoxy and para-chloro substituents showed moderate inhibitory effects on osteoclast formation. However, other PMSA-3 derivatives with an aryl ester group on the N-phenyl substituent did not show any inhibitory effect. Therefore, non-mesylated PMSA-3 derivatives showed a greater inhibitory effect than mesylated PMSA-3 derivatives. Among the non-mesylated PMSA-3 derivatives, 25-KR-03 as a methyl ester substituted derivative still showed the greater inhibitory effect than the aryl ester substituted derivatives (25-KR-20 to 27).

Therefore, 25-KR-03 was selected as a candidate to further evaluate the inhibitory effect on osteoclast formation.

### 2.2. Dose-dependent inhibition of RANKL/M-CSF-induced osteoclast formation

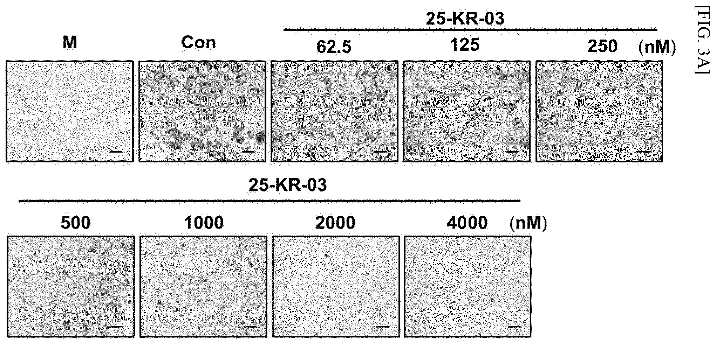

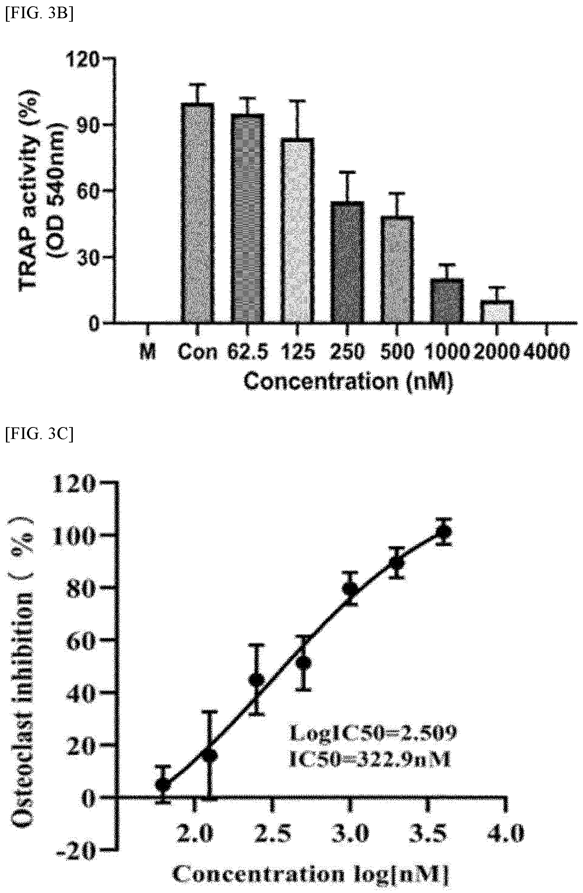

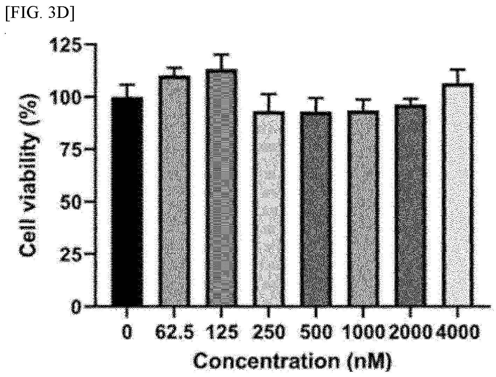

First, by adopting TRAP staining (a specific characteristic of osteoclasts) assay, the effect of 25-KR-03 to inhibit RANKL-induced osteoclast formation ('osteoclastogenesis') in the indicated concentration range (0, 62.5, 125, 250, 500, 1000, 2000, and 4000 nM) was confirmed. Differentiation of BMDM showed IC₅₀ of 322.9 nM and was significantly inhibited by 25-KR-03 (FIGS. 3A and 3B). Further, while 25-KR-03 had no significant effect on the cell viability of BMDMs below 4 µM, it completely blocked the formation of multinucleated mature osteoclasts (FIGS. 3A and 3D), indicating that 25-KR-03 inhibits RANKL-mediated osteoclastogenesis in a concentration-dependent manner without affecting cell viability in *vitro.*

Specifically, in FIG. 3A, the inhibitory effect of 25-KR-03 on osteoclast differentiation was shown using TRAP staining. BMMs were treated with 30 ng/mL M-CSF, 50 ng/mL M-CSF and mL rank; TRAP staining analysis was then performed to visualize osteoclastogenesis. PMSA-3-Ac was used as a control inhibitor. In FIG. 3B, TRAP activity of each group was measured at OD 540 nm. In FIG. 3C, the IC50 of 25-KR-03 on osteoclast differentiation is calculated and shown based on the TRAP activity of each group after challenge with the indicated doses of 25-KR-03. In FIG. 3D, cell viability of the indicated doses at 48 hours is shown as analyzed using a cell viability assay kit.

### 2.3. RANKL-induced F-actin belt formation and inhibition of bone resorption

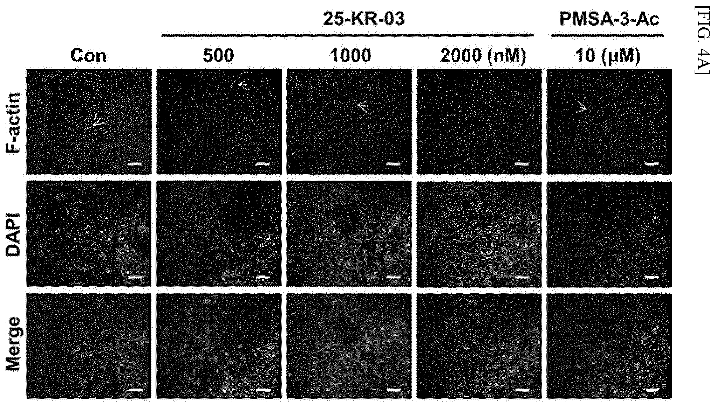

As shown in FIG. 4A, FITC-labeled phalloidin is used to mark the formed F-actin belts of mature osteoclasts. 25-KR-03 inhibited actin ring formation at the indicated doses, whereas the control group showed well-formed rings. Meanwhile, PMSA-3-Ac was used as a control inhibitor, and 25-KR-03 inhibited osteoclast F-actin belt formation more strongly than PMSA-3-Ac (10 µM).

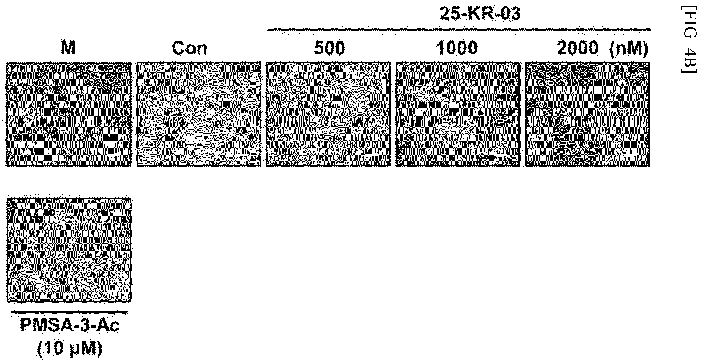

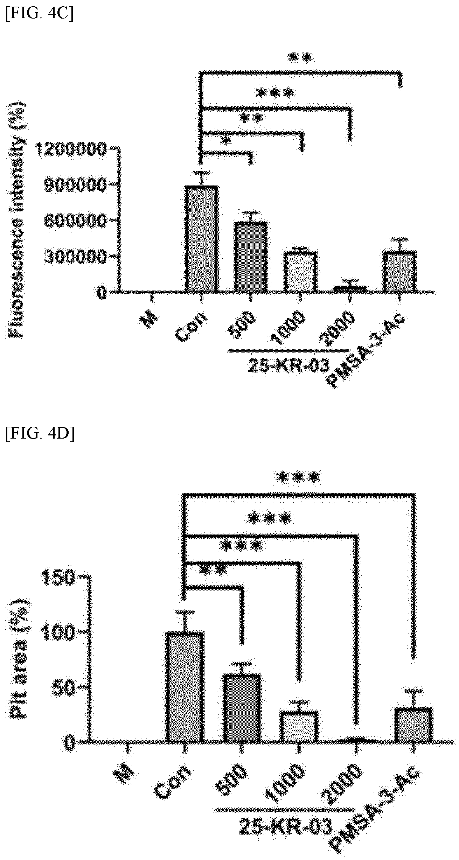

Bone resorption is an important function of osteoclasts in the bone remodeling process. Therefore, bone resorption analysis of 25-KR-03 was performed *in vitro* using fluoresceine amine-labeled calcium phosphate plates to determine the ability of 25-KR-03 to inhibit osteoclast bone resorption. As shown in FIGS. 4B and 4D, obvious bone resorption holes appeared in the groups treated with M-CSF and RANKL; the resorption pit area was significantly reduced by 25-KR-03 treatment (0.5, 1, and 2 µM). Further, 25-KR-03 reduced fluorescence intensity and pit area formation in a dose-dependent manner and was more effective than PMSA-3-Ac (FIGS. 3B to 3D). Collectively, these results suggest that 25-KR-03 significantly inhibits RANKL-induced F-actin ring formation and osteoclast-related bone resorption activity by reducing the formation of mature osteoclasts.

Specifically, actin ring formation was visualized during osteoclast formation in the presence of the groups indicated in FIG. 4A. FIG. 4B shows the results of BMDMs cultured on fluoresceine amine-labeled calcium phosphate plates and treated with the indicated doses of 25-KR-03 and PMSA-3-Ac for 6 days. In FIG. 4C, the fluorescence intensity of each group after 6 days was measured at excitation and emission wavelengths of 485 nm and 535 nm, respectively. The desalting area was calculated using ImageJ and is shown in FIG. 4D. All experiments were repeated three times.

### 2.4. Inhibition of expression of osteoclast differentiation genes during osteoclastogenesis

Due to RANKL and M-CSF stimulation, osteoclast-specific genes required for osteoclast formation and differentiation are induced. Therefore, several key genes mediating the differentiation and formation of multinucleated mature osteoclasts were analyzed using real-time PCR analysis.

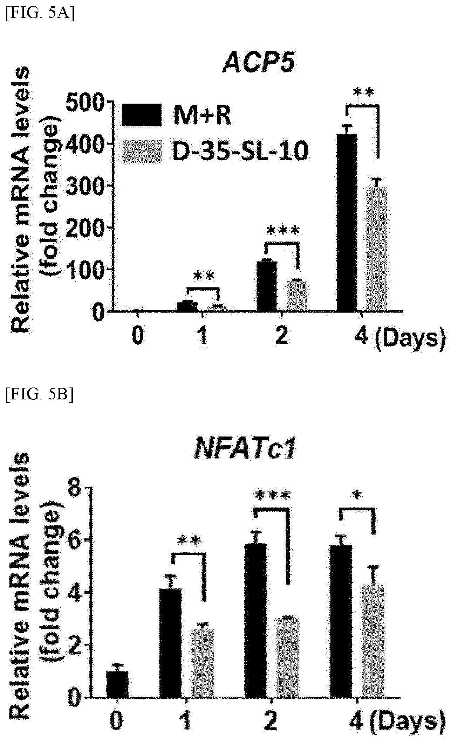

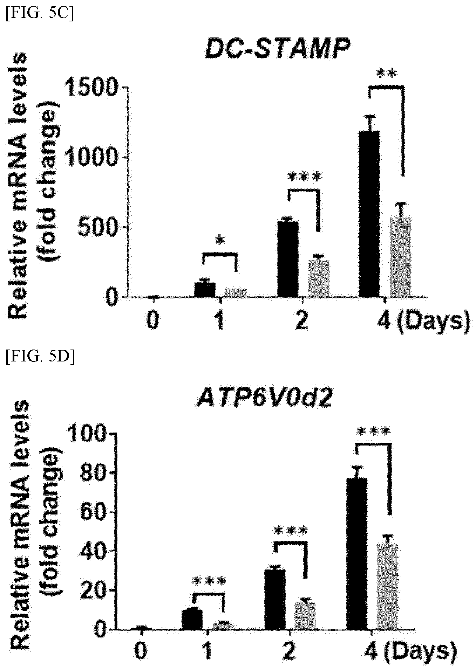

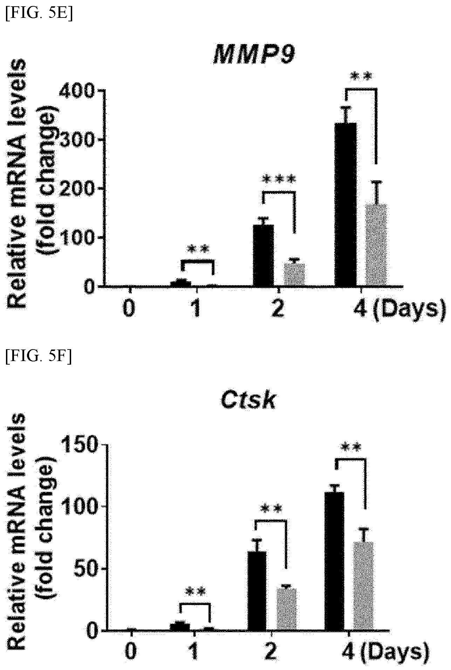

As shown in FIGS. 5A to F, stimulation with M-CSF and RANKL(Con) significantly induced transcription of the indicated osteoclast-specific genes (black bars), whereas, in the case of treatment with 25-KR-03, the relative mRNA expression levels of RANKL-induced osteoclastogenesis-related genes, including Acp5, NFATc1, DC-STAMP, ATP6V0d2, MMP9 and Ctsk (gray bars), were obviously decreased.

Specifically, FIGS. 5A to 5F show the results of measuring the relative mRNA levels of the indicated osteoclast-specific genes using qRT-PCR with or without 25-KR-03 treatment for 0, 1, 2, and 4 days. Black bars represent M-CSF and RANKL treatment groups ("Con") and gray bars correspond to 25-KR-03 treatment groups. Transcript levels were normalized to expression at day 0. All experiments were repeated three times.

### 2.5. RANKL/M-CSF-induced osteoclast differentiation through blocking NFATc1 nuclear translocation

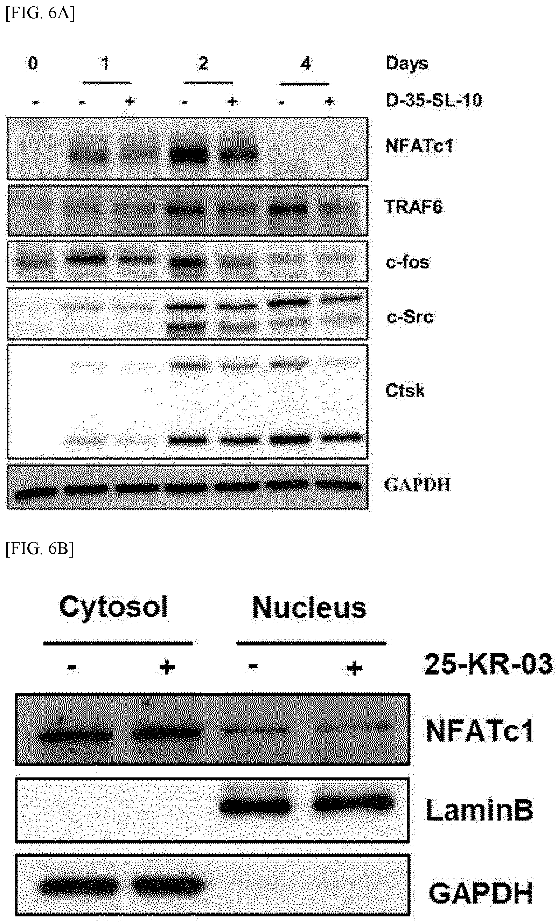

Due to the significant decrease in the expression of genes related to osteoclast formation with 25-KR-03 culture, several key proteins involved in the formation and activation of osteoclasts were investigated. As shown in FIG. 6A, Western blotting analysis suggested that 25-KR-03 had no significant effect on the protein expression of NFATc1, Traf6, c-src, GSK3β and calcineurin A, while blocking differentiation of osteoclasts compared to the control group. However, 25-KR-03 significantly inhibited the protein expression of the major reuptake protease Ctsk. Although NFATc1 protein levels were not significantly affected due to decreased transcription of genes required for osteoclast differentiation and decreased protein levels of Ctsk, it was speculated that 25-KR-03 inhibited osteoclastogenesis by affecting NFATc1 function.

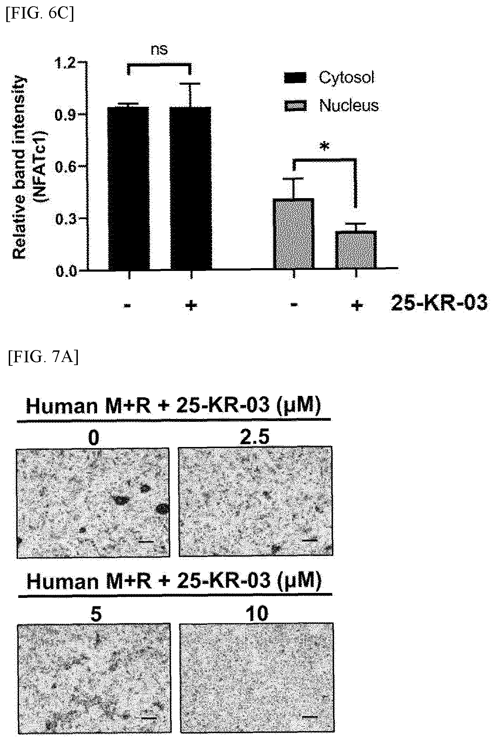

As known in the art, NFATc1 is dephosphorylated by calcineurin, which induces nuclear translocation of NFATc1 to transcribe target genes required for osteoclast differentiation upon RANKL stimulation, while Gsk3β rephosphorylates NFATc1 to export NFATc1 from the nucleus to the cytoplasm. Accordingly, nuclear translocation of NFATc1 was detected: as shown in FIGS. 6B and 6C, compared to the group without 25-KR-03, incubation with 25-KR-03 slightly increased NFATc1 protein levels in the cytoplasm (although not significant), but significantly reduced NFATc1 protein levels in the nucleus, suggesting that the inhibition of osteoclastogenesis by 25-KR-03 is due to the ability of 25-KR-03 to reduce levels of NFATc1 translocated to the nucleus; subsequently, transcription of target genes required for osteoclast formation was reduced.

Specifically, in FIG. 6A, immunoblotting was performed for analysis. Culture was performed with or without 25-KR-03 for the indicated days in induction medium. FIG. 6B shows the results of treating BMDMs with 25-KR-03 for 48 hours and analyzing the protein levels of NFATc1 in the cytoplasm and nucleus using immunoblotting. GAPDH/LaminB1 was used as a loading control. FIG. 6C shows the result of calculating the relative band intensity of FIG. 6B.

### 2.6. Attenuated hRANKL/M-CSF - Induction of differentiation of human IPSC-derived macrophages into mature osteoclasts

The above results suggest that 25-KR-03 has significant inhibitory effects on RANKL-induced osteoclast differentiation using BMDM. However, it is unclear whether 25-KR-03 has an inhibitory effect on human osteoclast differentiation. Accordingly, a human osteoclast differentiation model was established using human IPSC-derived macrophages. Cells were incubated with 50 ng/mL human M-CSF and 100 ng/mL RANKL to induce mature osteoclast formation.

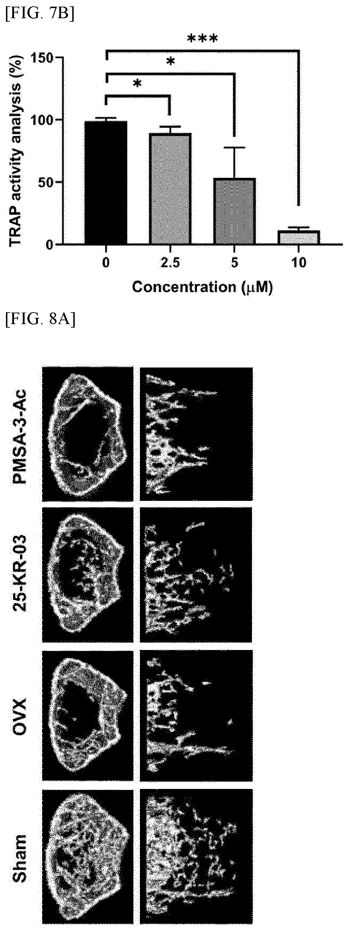

Specifically, in FIG. 7A, the inhibitory effect of 25-KR-03 on human osteoclast differentiation was analyzed through TRAP staining. Human IPSC-derived macrophages were treated with the indicated concentrations of 25-KR-03 for 10 days in the induction medium containing 50 ng/mL hM-CSF and 100 ng/mL hRANKL. After the formation of multinucleated osteoclasts, TRAP staining was performed to visualize the formed osteoclasts. FIG. 7B shows the analysis of TRAP activity in each group. These results indicate that 25-KR-03 has bioactivity in both mouse and human cells.

### 2.7. Inhibition of bone loss in ovariectomy-induced osteoporosis rat model

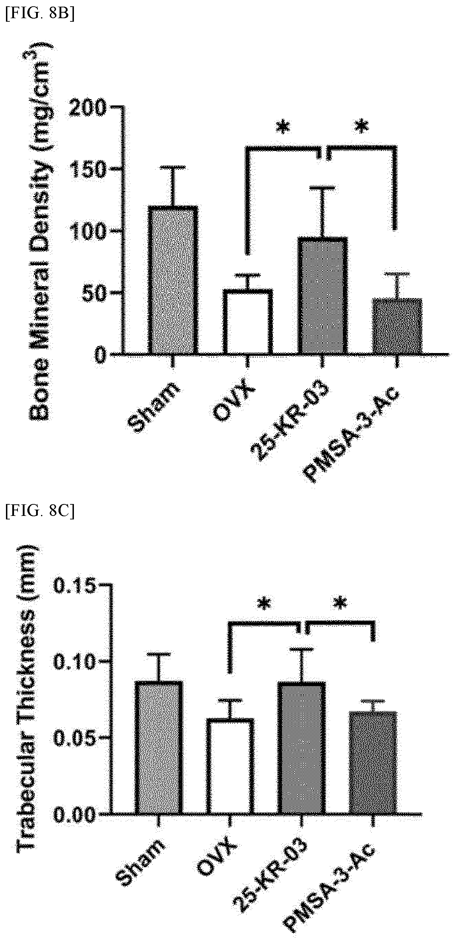

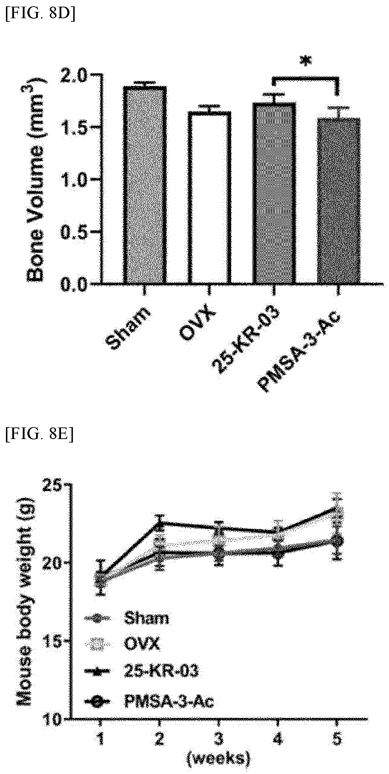

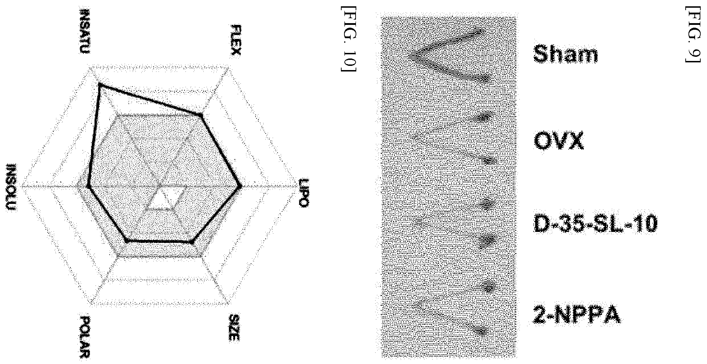

To further confirm the preventive role of 25-KR-03 *in vivo*, a mouse model of OVX-induced osteoporosis was established (FIGS. 8A and 8B). Images of uterine tissues from the sham, OVX, 25-KR-03, and PMSA-3-Ac groups are shown in FIG. 9, confirming the successful establishment of the osteoporosis model. FIG. 8C shows the weekly body weight of each group. There was no significant weight reduction after 4 weeks of treatment with 20 mg/kg of 25-KR-03, suggesting low or no apparent toxicity of 25-KR-03. Mouse femurs were scanned using micro-CT (FIG. 8A). 3D imaging analysis of the femur suggests slight but significant prevention of bone loss in OVX-induced osteoporosis mice compared with the OVX group, as indicated by Tb.Th and BMD values after treatment with 25-KR-03 for 4 weeks after surgery (FIGS. 8A and 8B).

However, when PMSA-3-Ac 20mg/kg was administered for 4 weeks after surgery, there was no significant effect compared to the OVX group, which is consistent with previous reports. In summary, these results show that 25-KR-03 is more effective in preventing osteoporosis than PMSA-3-Ac.

Specifically, FIG. 8A shows representative micro-CT 3D reconstructed image. In FIG. 8B, BMD, Tb.Th, and BV of each sample were measured and calculated. FIG. 8C shows the body weight of mice in each group for *in vivo* analysis. In the drawings, * *p* < 0.05 indicates calculated value of vs. OVX group or PMSA-3-Ac treated group.

### 3. ADME analysis

The results of ADME (Absorption, Distribution, Metabolism, and Excretion) analysis of 25-KR-03 using the SwissADME database are shown in Table 4 and FIG. 10 below.

**[TABLE 4]**

| Lipophilicity | |
|---|---|
| Log P_{o/w}(iLOGP) | 3.73 |
| Log P_{o/w}(XLOGP3) | 4.73 |
| Log P_{o/w}(WLOGP) | 4.29 |
| Log P_{o/w}(MLOGP) | 3.92 |
| Log P_{o/w}(SILICOS-IT) | 3.87 |
| Consensus Log P_{o/w} | 4.11 |

| Hydrophilicity | |
|---|---|
| Log S (ESOL) | -5.13 |
| Solubility | 2.88e-03 mg/ml; 7.33e-06 mol/l |
| Class | Moderately soluble |
| Log S (Ali) | -6.41 |
| Solubility | 1.54e-04 mg/ml ; 3.92e-07 mol/l |
| Class | Poorly soluble |
| Log S (SILICOS-IT) | -8.00 |
| Solubility | 3.90e-06 mg/ml ; 9.93e-09 mol/l |
| Class | Poorly soluble |

| Pharmacokinetics | |
|---|---|
| GI absorption | High |
| BBB permeant | No |
| P-gp substrate | No |
| CYP1A2 inhibitor | Yes |
| CYP2C19 inhibitor | Yes |
| CYP2C9 inhibitor | Yes |
| CYP2D6 inhibitor | Yes |
| CYP3A4 inhibitor | Yes |
| Log Kₚ(skin permeation) | -5.34 cm/s |

| Drug similarity | |
|---|---|
| Lipinski | Yes; 0 violation |
| Ghose | Yes |
| Veber | Yes |
| Egan | Yes |
| Muegge | Yes |
| Bioavailability score | 0.55 |

| Medical chemistry | |
|---|---|
| PAINS | 0 alert |
| Brenk | 0 alert |
| Leadlikeness | No; 3 violations: MW> 350, Rotors>7, XLOGP3>3.5 |
| Synthetic accessibility | 2.77 |

## Claims

1. A pharmaceutical composition for preventing or treating bone disease caused by hyper-differentiation of osteoclasts, comprising a compound represented by Formula 1 below or a pharmaceutically acceptable salt thereof: 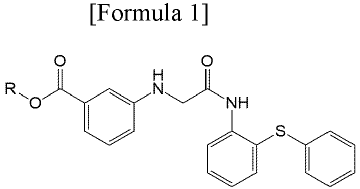 wherein R is a C₁₋₅ alkyl group.

2. The pharmaceutical composition according to claim 1, wherein R is a methyl group.

3. The pharmaceutical composition according to claim 1, wherein the bone disease is at least one selected from the group consisting of a fracture, osteoporosis, rheumatoid arthritis, periodontitis, Paget's disease, osteomalacia, osteopenia, bone atrophy, osteoarthritis, and avascular femoral necrosis.

4. A health functional food for preventing or treating bone disease caused by hyper-differentiation of osteoclasts, comprising a compound represented by Formula 1 below or a pharmaceutically acceptable salt thereof: 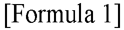 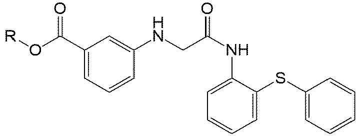 wherein R is a C₁₋₅ alkyl group.

5. The health functional food according to claim 4, wherein the bone disease is at least one selected from the group consisting of a fracture, osteoporosis, rheumatoid arthritis, periodontitis, Paget's disease, osteomalacia, osteopenia, bone atrophy, osteoarthritis, and avascular femoral necrosis.
